# EUROPEAN PATENT SPECIFICATION

(11) **EP 2 185 595 B1**
(45) Date of publication and mention of the grant of the patent: **10.12.2014**
(21) Application number: 08773968.6
(22) Date of filing: 10.07.2008
(51) Int. Cl.: C07K 16/28, C07K 16/30, C07K 16/46

(54) **RECOMBINANT, SINGLE-CHAIN, TRIVALENT TRI-SPECIFIC OR BI-SPECIFIC ANTIBODY DERIVATIVES**
REKOMBINANTE, TRIVALENTE, TRISPEZIFISCHE ODER BISPEZIFISCHE ANTIKÖRPERDERIVATE MIT EINER KETTE
DÉRIVÉS D'ANTICORPS BISPÉCIFIQUES OU TRISPÉCIFIQUES TRIVALENTS, À CHAÎNE UNIQUE, RECOMBINANTS

(30) Priority: 10.07.2007 EP 07013510
(43) Date of publication of application: 19.05.2010
(73) Proprietor: Friedrich-Alexander-Universität Erlangen-Nürnberg, 91054 Erlangen (DE)
(72) Inventor: KELLNER, Christian, 91207 Lauf (DE); SINGER, Heiko, 91094 Langensendelbach (DE); FEY, Georg, H., 91077 Neunkirchen a. Br. (DE); BRÜNKE, Jörg, 90480 Nürnberg (DE)
(74) Representative: Vossius & Partner
(86) International application number: PCT/EP2008/005654
(87) International publication number: WO 2009/007124

(56) References cited:
- EP-A- 1 314 741
- WO-A-01/85795
- WO-A-2004/106381
- BRUENKE JOERG ET AL: "Effective lysis of lymphoma cells with a stabilised bispecific single-chain Fv antibody against CD19 and Fc gamma RIII (CD16)" BRITISH JOURNAL OF HAEMATOLOGY, vol. 130, no. 2, July 2005 (2005-07), pages 218-228, XP002460816 ISSN: 0007-1048
- SEGAL D M ET AL: "Bispecific antibodies in cancer therapy" CURRENT OPINION IN IMMUNOLOGY, CURRENT BIOLOGY LTD, vol. 11, no. 5, 1 October 1999 (1999-10-01), pages 558-562, XP004257585 ISSN: 0952-7915 cited in the application
- PEIPP M ET AL: "Bispecific antibodies targeting cancer cells" BIOCHEMICAL SOCIETY TRANSACTIONS, COLCHESTER, ESSEX, GB, vol. 30, 2002, pages 507-511, XP002981328 ISSN: 0300-5127
- KIPRIYANOV S M ET AL: "Bispecific CD3 x CD19 diabody for T cell-mediated lysis of malignant human B cells" INTERNATIONAL JOURNAL OF CANCER, NEW YORK, NY, US, vol. 77, no. 5, 31 August 1998 (1998-08-31), pages 763-771, XP002115487 ISSN: 0020-7136
- WALZ ANNETTE ET AL: "Antitumour effects of a bispecific trivalent antibody in multicellular tumour spheroids" ANTICANCER RESEARCH, vol. 24, no. 2B, March 2004 (2004-03), pages 887-893, XP009092828 ISSN: 0250-7005

## Description

The present invention relates to a nucleic acid molecule encoding a polypeptide as characterised in the appended claims. The present invention furthermore relates to a vector comprising the nucleic acid molecule of the invention, a non-human host transformed with the vector of the invention, a method of producing a polypeptide comprising culturing the host cell of the invention under suitable conditions and isolating the polypeptide produced and a polypeptide encoded by the nucleic acid molecule of the invention or produced by the method of the invention. In addition, the present invention relates to diagnostic and pharmaceutical compositions and methods for treating tumours.

In this specification, a number of documents are cited. The disclosure content of these documents including manufacturers' manuals is herewith incorporated by reference in its entirety.

Leukaemias are cancers of the blood or bone marrow and characterized by an abnormal proliferation of blood cells, usually leukocytes. Leukaemia is clinically and pathologically split into its acute and chronic forms. *Acute leukaemia* is characterized by the rapid proliferation of immature blood cells. This high number of immature cells makes the bone marrow unable to produce healthy blood cells. Acute forms of leukaemia can occur in children and young adults. Immediate treatment is required in acute leukaemias due to the rapid progression and accumulation of the malignant cells, which then spill over into the bloodstream and spread to other organs of the body. *Chronic leukaemia* is distinguished by the excessive build up of relatively mature, but still abnormal, blood cells. Typically taking months to years to progress, the cells are produced at a much higher rate than normal cells, resulting in many abnormal white blood cells in the blood. Chronic leukaemia mostly occurs in older people, but can theoretically occur in any age group. Whereas acute leukaemia must be treated immediately, chronic forms are sometimes monitored for some time before treatment to ensure maximum effectiveness of therapy. The diseases are further classified according to the type of abnormal cell found most in the blood (lymphoid cells vs. myeloid cells) resulting in four main categories of leukaemia: Acute lymphocytic leukaemia (also known as Acute Lymphoblastic Leukaemia, or ALL), Chronic Lymphoid Leukaemia (CLL), Acute Myelogenous Leukaemia (also known as Acute Myeloid Leukaemia, or AML) and Chronic Myelogenous Leukaemia (CML).

The commonly applied therapy of cancerous diseases originating from the haematopoietic system includes irradiation and/or chemotherapy. Furthermore, under certain circumstances, the additional possibility of bone marrow transplantation is regarded suitable. However, these therapies are more or less toxic to the patient and most of the time, they do not lead to complete cure from the disease. Recently, an immunotherapy based on monoclonal antibodies has entered the clinical phase and already proven to be efficient. Prominent examples are Rituximab (anti-CD20) for the treatment of certain non-Hodgkin B-cell lymphomas (NHL) and Campath 1H (anti-CD52) in the treatment of certain chronic lymphatic leukaemias (Reff et al., 1994; Onrust et al., 1999; Riechmann et al.,1988; Hale et al., 1988). This approach has also proven to be successful in diseases such as different kinds of haemoblastosis and also solid tumours. Other antibodies are in an advanced clinical state, e.g. Epratuzumab (anti-CD22) for NHLs (Carnahan et al., 2003; Leonard et al.,2003).
An alternative to those antibodies are antibodies attached to radioactive isotopes. Examples of these antibodies are BEXXAR (anti CD20-I131) and ZEVALIN (anti CD20-Y90) (Kaminski et al., 1993; Davis et al., 2007; Cheung et al.; 2006). Instead of radioisotopes, other substances such as toxins may be used, such as done for the immunotoxin Mylotarg (anti CD33 coupled to the toxin calicheamicine; Hamann et al., 2002; Sievers et al., 1999).
General advantages of antibodies in the treatment of cancerous diseases are their higher specificity for cancerous cells as compared to healthy cells. In particular in the case of haematologic diseases, the tumour cells are easily accessible due to their presence in the blood, bone marrow or less compact tissues.
Major drawbacks of antibodies in the above use are their size which limits their accessibility to certain tissues or their ability to penetrate tumours. Furthermore, interactions with Fc receptors of non-cytotoxic cells (e.g. B cells or platelets) or with inhibitory receptors such as FcγRIIIb impairs their therapeutic effect (Peipp and Valerius, 2002; Clynes et al., 2000). In addition, bi-allelic polymorphisms can reduce the sensitivity to an antibody therapy, which was e.g. shown for Rituximab (Cartron et al., 2002; Weng and Levy, 2003).
While the clinical relevance of complement-dependent cytotoxicity still remains unclear (Weng and Levy 2001), the recruitment of immune effector cells and induction of ADCC by engagement of Fc receptors (FcRs) plays an important role *in vitro* and *in vivo,* as demonstrated for Rituximab (Cartron, *et al* 2002, Weng and Levy 2003). Therefore, enhancing ADCC activity may represent a promising approach to improve Ab-based therapeutics. Consequently, several different approaches have been used to enhance the effector cell mediated functions of different monoclonal antibodies (mAbs), e.g., improvement of the binding of the Fc-portion to the FcRs by glyco-engineering or by introduction of amino acid substitutions (Barbin, *et al* 2006, Lazar, *et al* 2006).
An additional significant improvement of effector functions was achieved through the generation of bispecific antibodies (bsAbs), which in some cases produced even stronger *in vitro* cytotoxicity than the parental mAbs (Weiner, *et al* 1993) and were able to overcome some of the limitations of conventional antibodies (Peipp and Valerius, 2002). Two binding sites, one for an antigen expressed on tumour cells and the other for a trigger molecule on the surface of immune effector cells (e.g. T cells, NK cells, monocytes, macrophages or granulocytes) lead to the effective recruitment of these effector cells to the tumour cells and to specific killing of the tumour cells via an antibody dependent cellular cytotoxicity (ADCC) or phagocytosis. Typically, the tumour cell lysis is induced by binding of the antibody to cytotoxic trigger molecules such as CD2, CD28 or CD3 (expressed on T cells), CD16 (FcγRIIIa, expressed on NK cells), CD64 (FcγRI; expressed on activated neutrophils and monocytes/macrophages) or CD89 (FcαRI, expressed on neutrophils; Peipp and Valerius, 2002). NK cells are known to be among the first to regenerate after stem cell transplantations, which makes them particularly suitable to eliminate cells of the minimal residual disease (MRD) (Eyrich et al., 2001; Lang et al., 2003).
Compared to previously utilized techniques, the availability of recombinant antibodies greatly facilitates the production and purification of bi-specific antibodies. Commonly known antibody formats are diabodies, minibodies, single-chain diabodies or tandem bi-specific single-chain fragments variable (bsscFv) (Peipp and Valerius, 2002), the two latter of which consist of only one molecule.

The antigen(s) chosen to be bound by the therapeutic antibody derivative is/are crucial for the therapy. Antibodies binding to CD19 expressed on B-cells have been described before in the treatment of B lymphoid neoplasias (Grossbard et al., 1992). CD19 is expressed on B cells in all stages of their development except for the mature plasma cell. Until now, this antigen has not been found on haematopoietic stem cells or other cells beyond the B lymphoid compartment. The formats used so far comprised whole antibodies, immunoconjugates, immunoliposomes, immunotoxins and bi-specific formats. The treatment of six patients with a murine CD19 IgG2A antibody led to a partial remission in one patient (Hekmann et al., 1991). A glycol-engineered chimeric CD19 antibody was capable of mediating ADCC via NK cells (Barbin et al., 2006), a further chimeric CD19 antibody decelerated the growth of a CD19-positive tumour in a SCID-mouse model (Pietersz et al., 1995). Anti-CD19 antibody derivatives coupled to the tyrosine kinase inhibitor genistein as well as to radionuclides, cytotoxins or exotoxins were developed which all showed an amelioration of the cancerous condition (Messinger et al., 1998; Vervoordeldonk et al., 1994; Vallera et al., 2004; Schwemmlein et al., 2007). Bi-specific antibodies known are e.g. those directed against CD19 and CD64 using interferon-stimulated macrophages as effector cells (Ely et al., 1996). Antibody derivatives directed against CD19 and CD3 (Haagen et al., 1992; Weiner and de Gast, 1995; Kiprianov et al, 1998; Loffler et al., 2000) as well as CD19 and CD16 (Kiprianov et al., 2002) activate cytotoxic T-cells or NK cells, respectively. Although cell culture data demonstrated significant lytic activity for the [CD19xCD3] bi-specific antibody derivatives, the cross-linking of CD3 mediated by the antibody led to nonspecific T-cell activation, causing a toxicity to the cell associated with the antibody (Segal et al., 1999). [CD19xCD16] antibody derivatives have been produced as single-chain Fv molecule, as double-chain diabody and as tetravalent diabody and have been proven to be effective in a mouse model of a human lymphoma (EP 1314741; Affimed Therapeutics AG). CD16 is the low affinity receptor for IgG (FcγRIII), which is constitutively expressed as a transmembrane isoform on the surface of NK cells and macrophages (CD16a), and as a glycosyl phosphatidyl inositol (GPI)-anchored molecule on the surface of neutrophils (CD16b) (Ravetch and Kinet, 1991; van deWinkel and Anderson, 1991). For intracellular signalling, CD16a requires association with the FcRγ chain containing the immunoreceptor tyrosine-based activation motif (ITAM), which triggers downstream signalling. Studies with conventionally coupled bi-specific antibodies redirecting NK cells via CD16 demonstrated potent cytolysis of cultured malignant cells and in animal models (Garcia de Palazzo et al, 1992; Hombach et al, 1993; Kipriyanov et al, 2002). Therefore, clinical trials with CD16-directed bi-specific antibodies were initiated (Weiner et al, 1995; Hartmann et al, 1997). However, immunogenicity of hybrid-hybridoma antibodies, as well as undesired side effects caused by the presence of Fc-domains, and difficulties in producing sufficient amounts of clinical-grade material limited these clinical trials. Another bi-specific anti[CD19xCD16] antibody utilizes two single chain Fvs combined in one polypeptide chain (Bruenke et al., 2005).

The effectiveness of antibodies depends on their stability in human serum, their location in the organism and their affinity. Several means have been developed to influence these properties. For example, a disulfide bridge between the two variable domains of an scFv antibody can greatly improve the serum stability but very often results in insoluble expression of the recombinant protein or leads to a reduction of affinity. Further measures applicable for improvement of pharmacokinetical properties are to couple the antibody derivative to polyethyleneglycol (PEG), components binding to human serum albumin (HSA), to HSA itself or to peptides providing N-glycosylation sites to the antibody derivative (Kubetzko et al, 2006; Huhalov & Chester, 2004; Muller et al, 2007; Schoonjans et al, 2000; Stork et al, 2008)
Finally, the affinity to the targeted tumour cells could be enhanced by developing multivalent antibody derivatives. Published formats comprise minibodies, tribodies, tetravalent diabodies or trivalent triabodies, which are equipped with one or more specificities. Their reduced size allows for tumour penetration as well as for a longer retention time in the organism since their molecular weight lies still above the exclusion barrier of the kidneys. One remaining disadvantage of the molecules comprising more than two antigen binding sites is that they still consist of at least two polypeptide chains since until now it has not been described that a single-chain molecule with more than two specificities could be obtained which is applicable as a therapeutic. If an antibody derivative consists of more than one polypeptide chain it is more difficult to produce and purify this derivative to obtain a homogenous mixture.
Finally, the similar affinity of antibody derivatives with the same number of specific binding sites for both tumour cells and effector cells often leads to the activation of the latter without being recruited to a tumour or other cell before, which leads to a reduced cytotoxic potential of the effector cell.

The present invention provides novel and advantageous antibody derivatives suitable as therapeutic in haematologic diseases having a higher affinity for tumour cells than for effector cells thus shifting the avidity towards tumour cells, being more stable and exhibiting a prolonged plasma retention time *in vivo.*

Accordingly, the present invention relates to a nucleic acid molecule encoding a polypeptide as characterised in the appended claims, wherein the polypeptide consists of (a) a first immunoglobulin domain consisting of a V_{L} domain linked to a V_{H} domain, wherein the immunoglobulin domain specifically binds to an antigen expressed on tumour cells; (b) a second immunoglobulin domain consisting of a V_{L} domain linked to a V_{H} domain, wherein the immunoglobulin domain specifically binds to an antigen expressed on tumour cells; and (c) a third immunoglobulin domain consisting of a V_{L} domain linked to a V_{H} domain, wherein the immunoglobulin domain specifically binds to an effector cell antigen, wherein the effector cell is selected from the group consisting of NK cells, T cells, neutrophilic granulocytes, monocytes and macrophages; wherein at least one of the antigens expressed on tumour cells and bound by the immunoglobulin domain of (a) or (b) is an antigen expressed on tumour stem cells or on tumour precursor or progenitor cells; and (d) optionally, at least one (poly)peptide unrelated to immunoglobulin domains; and wherein, the nucleic acid molecule encodes at least one linker which separates at least one V_{H} from a V_{L} domain or at least one V_{L} from a V_{H} domain.
In accordance with the present invention the term "nucleic acid molecule" defines a linear molecular chain consisting of more than 30 nucleotides. The group of molecules here designated as "nucleic acid molecules" also comprises complete genes.

"Nucleic acid molecules", in accordance with the present invention, include DNA, such as cDNA or genomic DNA, and RNA. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art.

The present invention furthermore contemplates nucleic acid molecules complementary to the above-defined nucleic acid molecule as well as nucleic acid molecules hybridizing thereto under stringent conditions.

It is well known in the art how to perform hybridization experiments with nucleic acid molecules. Correspondingly, the person skilled in the art knows what hybridization conditions she/he has to use to allow for a successful hybridization. The establishment of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985).
"Stringent conditions" refers to hybridization conditions under which the polynucleotides that are capable of hybridizing to the polynucleotides of the invention or parts thereof hybridize to these target sequences to a detectably greater degree than to other sequences (e.g., at least 2-fold over background). Stringent conditions are sequence-dependent and will be different in different circumstances. By controlling the stringency of the hybridization and/or washing conditions, target sequences that have at least 90% sequence identity, more preferably 95%, such as 98% and more preferred 100% sequence identity to the probe can be identified (highly stringent hybridization conditions). Alternatively, stringency conditions can be adjusted to allow a higher degree of mismatching in sequences (low stringency conditions of hybridization). Such highly stringent and low stringent conditions for hybridization are well known to the person skilled in the art.
For example "stringent conditions" refers to hybridization conditions which comprise, e.g. an overnight incubation at 65°C in 4x SSC (600 mM NaCl, 60 mM sodium citrate) followed by washing at 65°C in 0.1x SSC for one hour. Alternatively, hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. Said conditions for hybridization are also known by a person skilled in the art as "highly stringent conditions for hybridization".
Also contemplated are nucleic acid molecules that hybridize to the polynucleotides of the invention at lower stringency hybridization conditions ("low stringency conditions for hybridization"). Changes in the stringency of hybridization are primarily accomplished through the manipulation of formamide concentration (lower percentages of formamide result in lowered stringency), salt conditions, or temperature. For example, lower stringency conditions include an overnight incubation at 50°C in 4x SSC or an overnight incubation at 37°C in a solution comprising 6x SSPE (20x SSPE = 3M NaCl; 0.2M NaH₂PO₄; 0.02M EDTA, pH 7.4), 0.5% SDS, 30% formamide, 100 mg/ml salmon sperm blocking DNA; followed by washes at 50°C with 1x SSPE, 0.1% SDS. In addition, to achieve an even lower stringency, washes performed following stringent hybridization can be done at higher salt concentrations (e.g. 5x SSC). It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. The embodiment recited herein above preferably refers to highly stringent conditions and alternatively to conditions of lower stringency. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed).

The term "polypeptide" as used herein describes linear molecular chains of amino acids, including single chain proteins or their fragments, containing more than 30 amino acids. Accordingly, the term "peptide" as used in the present invention describes linear chains of amino acids containing up to 30 amino acids. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein", which are used interchangeably, also refer to naturally modified polypeptides/proteins where the modification is effected e.g. by glycosylation, acetylation, phosphorylation and similar modifications which are well known in the art.

The term "immunoglobulin domain comprising a V_{L} domain linked to a V_{H} domain" refers to single-chain fragment variable domains of immunoglobulins (scFv) which have been shown to be necessary and sufficient to bind their antigen. Each of the V_{H} or V_{L} domains comprises three complementarity determining regions (CDRs), highly variable regions mainly responsible for the binding of the antigen. Where applicable, a V_{L} or V_{H} domain may consist of at least one CDR per domain, preferably at least two, preferably all three, as long as the resulting immunoglobulin domain exerts the desired function, i.e. specifically binds to its target antigen.
The nucleic acid molecule of the invention may encode immunoglobulin domains derived from a single species, but also chimeric or humanized molecules.
As regards the arrangement of the V_{L} and V_{H} domain in the immunoglobulin domain, the V_{L} domain may be positioned N- or C-terminal of the V_{H} domain. Accordingly, in the nucleic acid of the present invention, the nucleic acid encoding the V_{L} domain may be positioned 5' or 3' of that encoding the V_{H} domain. The skilled person is able to determine which arrangement of the VH and VL domains is more suitable for a specific scFv.

The term "specifically binds " or "specifically binding" (having the same meaning as "specifically interacting") as used in accordance with the present invention means that the immunoglobulin domain does not or essentially does not cross-react with an epitope with a structure similar to that of the target antigen. Cross-reactivity of a panel of immunoglobulin domains under investigation may be tested, for example, by assessing binding of said panel of immunoglobulin domains under conventional conditions to the epitope of interest as well as to a number of more or less (structurally and/or functionally) closely related epitopes. Only those immunoglobulin domains that bind to the epitope of interest in its relevant context (e.g. a specific motif in the structure of a protein) but do not or do not essentially bind to any of the other epitopes are considered specific for the epitope of interest and thus to be immunoglobulin domains in accordance with this invention. Corresponding methods are described e.g. in Harlow and Lane, 1988 and 1999, loc cit.

The term "antigen expressed on tumour cells" refers to antigens expressed on the surface of tumour cells. "Tumour cells" in accordance with the present invention are transformed cells that are characteristic of or give rise to tumours. The term "tumour" in accordance with the present invention refers not only to solid tumours, but also to non-solid tumourous conditions such as, for example, leukaemia.

An "effector cell antigen" is an antigen expressed on the surface of effector cells. The term "effector cells" as used throughout the present invention refers to terminally differentiated leukocytes that perform one or more specific functions in the immune system. "Effector cells" in accordance with the present invention are NK cells, T cells, granulocytes, macrophages or monocytes. It is preferred that the effector cells are NK cells.

The term "stem cells" in accordance with the present invention refers to primal cells found in all multi-cellular organisms which retain the ability to renew themselves through mitotic cell division and can differentiate into a diverse range of specialized cell types. Haematopoietic stem cells (HSCs) are a small subset of bone marrow cells, representing approximately 1 : 10⁵ - 1 : 10⁶ cells, with the unique ability of generating all other mature cell types of the haematopoietic system. They have the defining characteristic ability to divide in both a symmetric and asymmetric manner, and to generate lineage committed progeny cells. HSCs rarely divide but have a high self-renewal capability. The lineage-committed daughter cells are the "progenitor" and "precursor cells", which proliferate more actively (Spangrude et al., 1988; Bryder et al., 2006). The term "precursor or progenitor cell" refers to immature or undifferentiated cells, typically found in post-natal animals. The term "tumour precursor or progenitor cells" in accordance with the present invention refers to cells with altered properties, e.g. regarding their proliferation behaviour or gene expression pattern, giving rise to tumour cells. Examples for such tumour precursor or progenitor cells are e.g. leukaemic precursor or progenitor cells. Progenitor cells share many common features with stem cells. Like stem cells, progenitor cells have a capacity for self-renewal and differentiation, although these properties may be limited compared with those of stem cells. Progenitor or precursor cells can be seen as intermediate progeny of stem cells still capable of proliferating.
The ratio of immunoglobulin domains binding to antigens expressed on tumour cells and those binding to effector cell antigens is an essential feature of the present invention. As discussed above, previously known antibody derivatives comprise bi-or tri- specific derivatives of multi-chain immunoglobulins or bi-specific derivatives of single-chain Fvs. To the best knowledge of the applicant, optimization of the ratio of binding sites for tumour antigens and effector cell antigens and its effect on the quality of the immune response against the tumour cells has so far not been published. The present invention for the first time discloses a ratio of 2:1 of immunoglobulin domains binding to tumour cell antigens and effector cells antigens. The higher fraction of immunoglobulin domains binding to antigens expressed on tumour cells has the effect that the affinity for the tumour cells is increased. Thus, the higher number of antigen binding moieties targeted to antigen expressed on tumour cells increases the probability that the polypeptide of the invention binds to a tumour cell before it binds to an effector cell. The recruitment of tumour cells prior to the effector cells is advantageous for the following reason: Up to now, in a therapeutic context, immune responses are induced upon binding of an immunoglobulin with one or two specificities or a natural trigger molecule to a respective antigen expressed on the surface of effector cells mediating an immune response. Thereby, the effector cells cannot distinguish whether an antibody molecule is bound to a tumour cell or not leading to an unspecific immune response in case the antibody derivative is not bound to a tumour cell. In contrast, the 2:1 ratio of antigen binding sites specific for tumour antigens and those specific for effector cells enhance the probability that the antibody derivative of the present invention binds to a tumour cell before it binds to an antigen of an effector cell. As a consequence of the enhanced avidity to the tumour cells the cell surface retention time on the tumour cells is prolonged resulting in an improved targeting potential as described above. This greatly reduces the amount of unspecific immune responses and may thus decrease adverse side-effects of previously known molecules.

Apart from the effects described above, the present invention for the first time demonstrates that a single chain polypeptide comprising three antigen binding specificities in the format of scFvs can be recombinantly and solubly expressed and exerts the desired effects. As described above, bi-specific single-chain Fv antibodies were known in the prior art. However, it was not generally believed that a tri-specific single-chain molecule which is supposed to be structurally complex, could be obtained which is correctly folded, stable and still has the desired function, i.e. it binds its target antigens, whereby the avidity for tumour cell antigens is higher than that for effector cells. Often, scFvs that had been fused to other protein domains, e.g. other antibody-derived fragments, had a lower affinity than the scFvs alone (Lu et al., 2002; Mc Call et al., 2001; Schwenkert et al., 2008). Thus it was anticipated that the central scFv, which is engaged at both its N- and C-terminus with another protein in the format of a scFv triple-body, would have a dramatically impaired affinity or functionality.
A further advantage of the format of the polypeptide of the present invention is that the combination of three scFvs in one polypeptide reduces the surface of the molecule. This and the lack of constant antibody domains reduces potential immunogenicity in the case of antibodies derived from other organisms than the one to be cured from a tumour.

In addition, bispecific antibody-derivatives in their simplest format, consisting of two scFvs connected by a flexible linker of approximately 10-20 amino acids, have a relative molecular mass (Mr) of only about 50-60 kDa, and proteins with Mr 65 kDa are rapidly cleared from the bloodstream by the kidneys (Kipriyanov et al, 1999; Huhalov & Chester, 2004). Rapid clearance from the blood results in poor retention at the tumour site (Hu et al, 1996). Consequently, the size of the bispecific proteins has been increased by various modifications, including PEGylation (Kubetzko et al, 2006), N-glycosylation or the addition of further protein domains (Huhalov & Chester, 2004; Muller et al, 2007; Schoonjans et al, 2000; Stork et al, 2008). These modifications resulted in improved plasma retention times and body retention times *in vivo* (Kontermann, 2005) which did, however, not improve the binding properties of the molecule to tumour cells as could be achieved with the molecules of the present invention.

In a preferred embodiment, the immunoglobulin domain of items (a) and (b) bind to the same antigen expressed on tumour cells. For example, both immunoglobulin domains may bind to CD7, CD19, CD33, CD13, CD44var, CD123, C-type lectin-like molecule-1 (CLL-1), CD96, the latter being present in leukaemia stem cells (LSCs) in AML, or Melanoma-associated Chondroitin Sulfate Proteoglycan (MCSP).

In another preferred embodiment, the immunoglobulin domain of items (a) and (b) bind to different antigens expressed on tumour cells.

In a further preferred embodiment, the at least one antigen expressed on tumour stem cells or tumour precursor or progenitor cells is CD19, CD33, CD13, CD44var, CD123, CLL-1 or CD96.

In an additional preferred embodiment of the present invention, the antigens expressed on tumour cells, preferably on tumour stem cells or tumour precursor or progenitor cells, and bound by the immunoglobulin domains of (a) and (b) are CD19 and CD33, CD19 and CD13, CD19 and CD123, CD19 and CD44var, CD19 and MCSP, CD33 and CD13, CD33 and CD123, CD33 and CD44var, CD33 and CLL-1, CD33 and CD96, CD13 and CD123, CD13 and CD44var, CD13 and CLL-1, CD13 and CD96, CD123 and CD44var, CD123 and CLL-1, CD123 and CD96, CD44var and CLL-1, CD44var and CD96, or CLL-1 and CD96.

In general, on healthy cells only one type of the above antigens is expressed at a time. However, in certain tumours, particularly in certain leukaemias, more than one of the above or other specific antigens may be expressed at the same time. Prominent examples described in the prior art are CD13 and CD19, CD33 and CD19, as well as CD19 and MCSP (Melanoma-associated Chondroitin Sulfate Proteoglycan), being co-expressed in bi-phenotypic or mixed lineage leukaemias (Greil *et al.,* 1994; Hrusak and Porwit-MacDonald, 2002; Casasnovas *et al.,* 2003; Haagen *et al.,* 1992). The polypeptide encoded by the nucleic acid molecule of the present invention and having a specificity for two different antigens usually not expressed simultaneously on healthy cells has an increased affinity for aberrant cells, i.e. tumour cells simultaneously expressing two of the above antigens This enhances the selectivity of the molecule for the tumour cells.

In another preferred embodiment, the effector cell antigen bound by the immunoglobulin domain of (c) is CD16 (FcγRIIIa) or CD64 (FcγRI). Further antigens suitable in the context of the present invention are CD89, NKG2D, NKp30, NKp44, NKp46, CD3, CD28 or CD2.

In a further preferred embodiment, at least one immunoglobulin domain comprises at least one cysteine residue capable of forming intramolecular disulfide bridges.

It is known that intramolecular cysteine bridges stabilize an antibody-fragment and positively influence its serum stability and/or in some cases its binding affinity for the target epitope etc. Disulfide bridges are generally found in naturally occurring antibodies, where they are present in the variable and constant regions. Many scFv antibodies were found to have a lower affinity for antigen than the Fab counterpart which was attributed to the fact that the peptide linker of the scFv interferes with binding (Batra et al., 1992; Pantoliano et al., 1991; Stemmer et al., 1993). In order to improve the performance of scFv antibodies with different numbers of binding sites, it was attempted to introduce cysteins capable of forming disulfide bridges at positions in the molecule where they do not naturally occur. The following prerequisites have to be met when designing a modified antibody with artificial cysteine bridges. First, the disulfide bridge should connect amino acids in structurally conserved regions of the Fv, i.e. in the V_{H} and V_{L} regions. Furthermore, the distance between the V_{H} and the V_{L} domain should be small enough to enable the formation of a disulfide bridge without the generation of adverse strain in the Fv molecule. Finally, the disulfide bridge should be distant enough from the CDRs to not interfere with antigen binding. As a consequence, cysteines introduced to form the disulfide bridge are in the framework regions of the Fv which connect the CDRs of the V_{H} and V_{L} regions at positions that are structurally conserved in most antibodies. Glockshuber et al. (1990), Brinkmann et al. (1993) and Reiter et al. (1994) set out these criteria and successfully established the first disulfide stabilized Fv antibodies.
However, most of the trials undertaken to stabilize antibodies comprising only variable regions in this way as in scFvs encountered various difficulties, i.e. similar problems were faced during expression of the modified antibodies as compared to the expression of recombinant whole antibodies. For example, the resulting molecules turned out to be insoluble when recombinantly expressed, which is probably due to uncontrolled disulfide bridge formation and aggregation resulting therefrom. Apart from that, disulfide stabilized scFvs also often displayed a reduced affinity to the antigen as compared to their non disulfide stabilized counterparts. The present invention could overcome this problem. Although predicted to result in similar difficulties as described above by various experts in the field, the immunoglobulin domains of the antibody derivative of the present invention could be stabilized by intradomain cysteine bridges without at the same time turning the whole molecule insoluble. Mutations in the amino acid sequence of the polynucleotide of the invention can e.g. be introduced according to Reiter et al. (1994) at base triplets at position 100 in the V_{L} domain or at position 44 in the V_{H} domain, so that they encode a cysteine residue. All positions are according to the Kabat numbering (Kabat et al., 1991).
In the context of the present invention, immunoglobulin domains binding to certain antigens are stabilized by mutating two appropriate amino acids as described above to cysteins. The introduction of disulfide bridges into immunoglobulin domains of scFvs specific for CD19 and CD16 was shown to greatly enhance the stability of the molecules, without affecting their specificity or reducing their affinity and functionality, which was observed by the present inventors for other scFvs specific for MCSP or CD7.
Means to introduce mutations into a nucleic acid molecule are known to the skilled person and can e.g. be retrieved from in Sambrook, 2001 and Ausubel, 2001.

In accordance with the present invention, the nucleic acid molecule of the invention encodes at least one linker which separates at least one V_{H} from a V_{L} domain or at least one V_{L} from a V_{H} domain.

The term "linker" as used in accordance with the present invention relates to a sequel of amino acids linking either the V_{H} and V_{L} domains of one immunoglobulin domain or two immunoglobulin domains. Thus, the linker may separate at least one V_{H} and V_{L} region forming one immunoglobulin domains and/or the linker may separate at least one V_{L} and V_{H} region of neighbouring immunoglobulin domains.

Linkers as envisaged by the present invention are (poly)peptide linkers of 1 to 40 amino acids in length. Preferably, the linkers are of 5 to 25 amino acids in length. Even more preferably, the linkers are 10 to 20 amino acids in length. The linkers separating the V_{H} and V_{L} domains of one immunoglobulin domain and the ones separating the different immunoglobulin domains can have the same or different lengths and may comprise the same or a different amino acid sequence. Preferably, the linkers have the same length and the same amino acid sequence.

In another preferred embodiment, the linkers separating the immunoglobulin domains differ in length and/or amino acid sequence from those separating the V_{H} and V_{L} regions within an immunoglobulin domain. For example, the former could be longer and designed in order to promote the flexibility of the immunoglobulin domains towards each other or to facilitate correct folding of the molecule and/or enhance the affinity of one immunoglobulin domain to its target antigen. Furthermore, the nature, i.e. the length and/or amino acid sequence of the linker may modify or enhance the stability and/or the solubility of the molecule.
The length and sequence of a linker depends on the composition of the V_{H} and V_{L} domains forming an immunoglobulin domain. For example, starting from the N-terminus of the polypeptide of the invention or from the 5'-end of the nucleic acid molecule of the invention, if the V_{L} domain is followed by the V_{H} domain, linkers of 20 amino acid separating the V regions of one immunoglobulin domain may be optimal. In contrast, if the V_{H} domain is followed by the V_{L} domain, the respective linker my have an optimal length of 15 amino acids. Without wishing to be bound by any scientific theory, it is believed that these differences may be due to sterical reasons leading to linkers of different lengths promoting the correct folding of immunoglobulin domains having a different arrangement of V domains.
The skilled person is well aware of methods to test the suitability of different linkers within or between immunoglobulin domains. For example, the properties of the molecule can easily be tested by comparing the binding affinity of the immunoglobulin domain. In case of the tri-specific molecules of the invention, the respective measurements for each immunoglobulin domain may be carried out. The stability of the resulting molecule can be measured - using a flow cytometry based method to determine the residual binding capacity of the molecule after incubation in human serum at 37°C for several time periods. Other suitable tests can e.g. be found in Bruenke et al. (2005). In a preferred embodiment, at least two variable domains are fused by a flexible linker using e.g. the amino acids alanine and serine or glycine and serine. Preferably the linker sequences are (Gly₄Ser)₄, or (Gly₄Ser)₃. In another preferred embodiment no linker is present between at least one V_{H} and V_{L} domain or V_{L} and V_{H} domain within or between immunoglobulin domains.

In another preferred embodiment, the nucleic acid molecule of the invention encodes two immunoglobulin domains specifically binding to CD19 and one immunoglobulin domain specifically binding to CD16 (FcγRIIIa).

In another preferred embodiment, the nucleic acid molecule of the invention encodes two immunoglobulin domains specifically binding to CD33 and one immunoglobulin domain specifically binding to CD16 (FcγRIIIa).

An antibody encoded by the nucleic acid molecule of the invention comprising two binding sites for a tumour antigen and one for the effecter antigen exerts superior properties as compared to the bi-specific scFv antibody derivative. The novel antibody derivative of the present invention exhibits in antibody dependent cellular cytotoxicity (ADCC) assays a higher efficacy as compared to the bi-specific scFv derivative. Furthermore, the single chain Fv triple-body (triabody) has more favourable pharmacokinetical properties. In mice, the in vivo serum retention half-life is higher as the half life displayed by the tandem bi-specific scFv antibody. Furthermore, a disulfide-stabilized antibody having disulfide bridges in all immunoglobulin domains was shown previously to be more stable as compared to a non disulfide-stabilized variant of the bi-specific scFv antibody (Bruenke *et al.,* 2005).

As shown in the examples, the recombinant antibody fragment [CD33xCD16xCD33] has a higher avidity for the myeloid tumour antigen CD33 than the bsscFv [33xds16] used as a CD33 monovalent control protein. Importantly, the higher avidity for CD33 translated into an increased ADCC potential: the single chain Fv triabody (sctb) [33xds16x33] needed 8-fold lower concentrations to induce half-maximum lysis as the bsscFv [33xds16].

In yet another preferred embodiment, the polypeptide further comprises at least one (poly)peptide unrelated to immunoglobulin domains which can be e.g. a tag or a functional (poly)peptide suitable to improve the performance of the polypeptide of the invention. The tag can e.g. be a Strep-tag, a His-tag, a Myc-tag or a Flag-tag. Functional polypeptide are e.g. a kappa secretion leader, human serum albumin (hsa) or fragments thereof, peptides capable of binding to hsa or other serum proteins; peptides capable of binding to neonatal Fc receptor (FcRn), human muscle aldolase (hma) or fragments thereof, CD8 hinge region, immunoglobulin constant regions, Interleukin-2, Interleukin-15 and Interleukin-18, Granulocyte-Macrophage-Colony Stimulating Factor (GM-CSF), Granulocyte Colony Stimulating Factor (G-CSF) or a peptide providing at least one N-glycosylation site.

The term "(poly)peptide" as used herein describes a group of molecules which comprises the group of peptides, consisting of up to 30 amino acids, as well as the group of polypeptides, consisting of more than 30 amino acids, as defined above.

The term "fragments thereof" in connection with the present invention refers to fragments of proteins still having one or more of the biological functions of the full-length (poly)peptide. In particular, the fragments of (poly)peptides as envisaged in the present invention are capable of increasing the stability and/or the serum half-life of the antibody derivative of the present invention.
It is well known in the art that functional polypeptides may be cleaved to yield fragments with unaltered or substantially unaltered function. Such cleavage may include the removal of a given number of N- and/or C-terminal amino acids. Additionally or alternatively, a number of internal (non-terminal) amino acids may be removed, provided the obtained polypeptide has the function of the full length (poly)peptide. Said number of amino acids to be removed from the termini and/or internal regions may be one, two, three, four, five, six, seven, eight, nine, ten, 15, 20, 25, 30, 40, 50 or more than 50. Any other number between one and 50 is also deliberately envisaged in accordance with this invention.
Means and methods for determining such functional domains of polypeptides are well known in the art and include experimental and bioinformatic means. Experimental means include the systematic generation of deletion mutants and their assessment in assays for the desired functions above known in the art. Bioinformatic means include database searches. Suitable databases included protein sequence databases. In this case a multiple sequence alignment of significant hits is indicative of domain boundaries, wherein the domain(s) is/are comprised of the/those subsequences exhibiting an elevated level of sequence conservation as compared to the remainder of the sequence. Further suitable databases include databases of statistical models of conserved protein domains such as Pfam maintained by the Sanger Institute, UK (www.sanger.ac.uk/Software/Pfam).

Some of the (poly)peptides to be further encoded by the nucleic acid molecule of the present invention may facilitate the purification of the recombinantly expressed polypeptide, e.g. various tags. Methods to add tags and/or other polypeptides to the polypeptide encoded by the nucleic acid molecule of the present invention are well known to the skilled person and described e.g. in Sambrook, 2001.

In a preferred embodiment, the tumour cells of the invention are preferably leukaemia or lymphoma cells. Depending on the origin and the stage of the lymphoid or myeloid cells the leukaemia originates from, different patterns of antigens are expressed on their surface. Antigens expressed on the surface of cells derived from the B-cell lineage are e.g. CD19, CD20 or CD22, whereas antigens typical for cells derived from myeloid cells are e.g. CD13, CD33 or CD123. These antigens are not exclusively expressed on leukaemia cells but also on healthy cells (Taussig et al., 2005).
The expression of CD19 on leukemic stem cells and/or progenitor cells from certain types of B-ALL has been reported (Castor et al., 2005). CD44v6 is expressed on a subset of both lymphoid and myeloid precursor cells and is strongly over-expressed on AML cells and AML leukaemia stem cells (LSCs) (Jin et al., 2006; Legras et al., 1998). CD13, CD33, CD96, CD 123 and CLL-1 are expressed on AML cells and LSCs of myeloid origin (Taussig et al., 2005; van Rhenen et al., 2007, Hosen et al., 2007).
In another preferred embodiment, the tumour stem cells are leukaemic stem cells. "Leukaemic stem cells" (LSC) in accordance with the present invention are a population of malignantly transformed cells, which drive the development of leukaemias in a similar manner as HSCs drive the generation of normal hematopoietic cells. They represent a small minority of all leukaemic cells (approx. 1 : 50,000 - 1 : 10⁶). Their defining properties are the ability of indefinite self-renewal and the ability to divide both in a symmetric and asymmetric manner. The sum of all leukemic cells in one patient is therefore not a homogeneous population of cells, but a heterogeneous mixture consisting of leukemic stem cells, intermediate progenitors and the bulk of more differentiated leukemic blast cells. The LSCs have a different composition of cell surface markers than the bulk leukaemia cells and are highly resistant to chemotherapeutics. They often survive chemotherapy, which eliminates the bulk of tumour cells and generates a remission. The remaining few LSCs are among the minimal residual disease (MRD) cells, which fuel the repopulation of the leukaemia mass ("relapse") (Tan et al., 2006; Bonnet and Dick, 1997; Lapidot et al., 1994; Passegue et al., 2003).

In another preferred embodiment the tumour precursor or progenitor cells are leukaemic precursor or progenitor cells.

In a further preferred embodiment, the tumour is a leukaemia or lymphoma. As described above, Leukaemias are cancers of the blood or bone marrow and are characterized by an abnormal proliferation of blood cells, usually leukocytes. Leukaemias can develop at different stages during development, including the stem cell stage (Castor et al., 2005).

In a different aspect, the present invention relates to a vector comprising the nucleic acid molecule of the present invention.

Preferably, the vector is a plasmid, cosmid, virus, bacteriophage or another vector used e.g. conventionally in genetic engineering.

The nucleic acid molecule of the present invention may be inserted into several commercially available vectors. Non-limiting examples include prokaryotic plasmid vectors, such as the pUC-series, pBluescript (Stratagene), the pET-series of expression vectors (Novagen) or pCRTOPO (Invitrogen) and vectors compatible with expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Intvitrogen).

The nucleic acid molecule of the present invention referred to above may also be inserted into vectors such that a translational fusion with another nucleic acid molecule is generated. The other nucleic acid molecules may encode a protein which may e.g. increase the solubility and/or facilitate the purifcation of the protein encoded by the nucleic acid molecule of the invention. Non-limiting examples include pET32, pET41, pET43 (Novagen). The vectors may also contain an additional expressible polynucleotide coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta®.

For vector modification techniques, see Sambrook and Russel, 2001. Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside.* ("IPTG"). For recombinant expression, the antibody fragment may be ligated between e.g. the PelB leader signal, which directs the recombinant protein in the periplasm and the gene III in a phagemid called pHEN4 (described in Ghahroudi *et al,* 1997). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). The co-transfection with a selectable marker such as dhfr, gpt, neomycin, hygromycin genes for eukaryotic cells or tetracycline, kanamycin or ampicillin resistance genes for culturing in *E. coli* and other bacteria allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using these markers, the mammalian cells are grown in selective medium and the cells with the highest resistance are selected.

Possible regulatory elements permitting expression in prokaryotic host cells comprise, e.g., the *lac, trp* or *tac* promoter, the lacUV5 or the trp promotor in *E*. *coli,* and examples for regulatory elements permitting expression in eukaryotic host cells (the more preferred embodiment) are the *AOX1* or *GAL1* promoter in yeast or the CMV- (Cytomegalovirus), SV40-, RSV-promoter (Rous sarcoma virus), the gai10 promoter, human elongation factor 1α-promoter, CMV enhancer, CaM-kinase promoter, the Autographa californica multiple nuclear polyhedrosis virus (AcMNPV) polyhedral promoter or a globin intron in mammalian and other animal cells. Preferred promoters are natural immunoglobulin promoters. Besides elements which are responsible for the initiation of transcription such regulatory elements may also comprise transcription termination signals, such as the SV40-poly-A site or the tk-poly-A site or the SV40, lacZ and AcMNPV polyhedral polyadenylation signals, downstream of the polynucleotide.

The coding sequences inserted in the vector can e.g. be synthesized by standard methods, or isolated from natural sources or produced semi-synthetically, i.e. by combining chemical synthesis and recombinant techniques. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, promoters, enhancers, and/or insulators), internal ribosomal entry sites (IRES) (Owens *et al.,* 2001) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Specifically-designed vectors allow the shuttling of DNA between different hosts, such as bacteria- fungal cells or bacteria-animal cells.
An expression vector according to this invention is capable of directing the replication, and the expression, of the polynucleotide and encoded enzyme of this invention. Suitable expression vectors which comprise the described regulatory elements are known in the art such as Okayama-Berg cDNA expression vector pcDV1 (Pharmacia), pRc/CMV, pcDNA1, pcDNA3 (In-Vitrogene, as used, inter alia in the appended examples), pSPORT1 (GIBCO BRL) or pGEMHE (Promega), or prokaryotic expression vectors, such as lambda gt11, pJOE, the pBBR1-MCS - series, pJB861, pBSMuL, pBC2, pUCPKS, pTACT1 or, preferably, the pET vector (Novagen).

The nucleic acid molecules of the invention as described herein above may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as eukaryotic expression system for the nucleic acid molecules of the invention. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook, 2001 and Ausubel, 2001.

In yet another aspect, the present invention relates to a non-human host transformed with the vector of the invention.

Suitable prokaryotic hosts comprise e.g. bacteria of the species Escherichia, *Bacillus, Streptomyces* and *Salmonella typhimurium.* Suitable eukaryotic host cells are e.g. fungal cells, inter alia, yeasts such as *Saccharomyces cerevisiae* or *Pichia pastoris* or insect cells such as Drosophila S2 and Spodoptera Sf9 cells and plant cells. Appropriate culture mediums and conditions for the above-described host cells are known in the art.
Mammalian host cells that could be used include, human Hela, HEK293, H9 and Jurkat cells, mouse NIH3T3 and C127 cells, COS 1, COS 7 and CV1, quail QC1-3 cells, mouse L cells, Chinese hamster ovary (CHO) cells and Bowes melanoma cells. Also within the scope of the present invention are primary mammalian cells such as mouse embryonic fibroblasts (MEF). Alternatively, the recombinant protein of the invention can be expressed in stable cell lines that contain the gene construct integrated into a chromosome.

In a more preferred embodiment, said cell is a primary cell or primary cell line. Primary cells are cells which are directly obtained from an organism. Suitable primary cells are, for example, mouse embryonic fibroblasts, mouse primary hepatocytes, cardiomyocytes and neuronal cells as well as mouse muscle stem cells (satellite cells) and stable, immortalized cell lines derived thereof.

Transgenic non-human animals as hosts transfected with and/or expressing the nucleic acid molecule of the present invention also lie within the scope of the invention. In a preferred embodiment, the transgenic animal is a mammal, e.g. a hamster, cow, cat, pig, dog or horse.

Furthermore, the present invention relates to a method for the production of a polypeptide comprising culture of the host cell of the invention under suitable conditions and isolation of the recombinant polypeptide produced.

Suitable conditions for culturing a prokaryotic or eukaryotic host are well known to the person skilled in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. *E*. *coli* can be cultured from 4 to about 37 °C, the exact temperature or sequence of temperatures depends on the molecule to be overexpressed. In general, the skilled person is also aware that these conditions may have to be adapted to the needs of the host and the requirements of the polypeptide expressed. In case an inducible promoter controls the nucleic acid of the invention in the vector present in the host cell, expression of the polypeptide can be induced by addition of an appriopriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycine. The cells can be kept at 37 °C in a 5% CO₂, water saturated atmosphere.
Suitable media for insect cell culture is e.g. TNM + 10% FCS or SF900 medium. Insect cells are usually grown at 27 °C as adhesion or suspension culture.
Suitable expression protocols for eukaryotic cells are well known to the skilled person and can be retrieved e.g. from in Sambrook, 2001.

Methods of isolation of the polypeptide produced are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001.

In another aspect, the present invention relates to a polypeptide encoded by the nucleic acid molecule of the invention or produced by the method of the invention.

In addition, the present invention relates to a diagnostic composition comprising at least one of the nucleic acid molecule of the invention, the vector of the invention or the polypeptide of the invention.

The enhanced affinity and/or avidity of the polypeptide of the invention enables for its use in diagnostic assays. For example, these molecules can be used as sensitive detection agents for malignant cells, expressing the respective antigens on the cell surface. Therefore, mono-specific variants of high avidity, which are equipped with three binding moieties directed against one cell surface antigen can be generated. As another example, tri-specific molecules can be useful for detection of cells expressing at least one of the three antigens the molecule is directed to, with one single agent. Especially the high stability of disulfide stabilized scFv fragments which are incorporated in this format allows a long time storage, which is desirable for diagnostic agents.
Furthermore, the present invention relates to a pharmaceutical composition comprising the nucleic acid molecule of the invention, the vector of the invention or the polypeptide of the invention.

In accordance with the present invention, the term "pharmaceutical composition" relates to a composition for administration to a patient, preferably a human patient. The pharmaceutical composition of the invention comprises the compounds recited above (wherein the term "compound" refers to the mentioned nucleic acid molecule, vector and the polypeptide as well as fragments or derivatives or modifications thereof), alone or in combination. It may, optionally, comprise further molecules capable of altering the characteristics of the compounds of the invention thereby, for example, stabilizing, modulating and/or activating their function. The composition may be in solid, liquid or gaseous form and may be, *inter alia*, in the form of (a) powder(s), (a) tablet(s), (a) solution(s) or (an) aerosol(s). The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutically acceptable carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. The term "parenteral" as used herein refers to modes of administration, which include intravenous, intramuscular, intraperitoneal, intrasternal, subcutaneous and intraarticular injection and infusion. These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

As mentioned above, the nucleic acid molecule of the invention may be formulated into a pharmaceutical composition. The nucleic acid molecule is eventually to be introduced into the desired cells. Appropriate formulations include those wherein 10⁶ to 10¹² copies of the DNA molecule, advantageously comprised in an appropriate vector are administered per dose. The vector may be, for example, a phage, plasmid, viral or retroviral vector. Retroviral vectors may be replication competent or replication defective. In the latter case, viral propagation generally will occur only in complementing host/cells.

The nucleic acid molecule of the invention, the vector of the invention or the polypeptide of the invention can be used for the treatment of tumours.

The desired effect in connection with the use in treatment is inducing an immune response against cells expressing the target tumour antigen(s), especially leukaemia cells.

In a preferred embodiment, the tumours to be treated are leukaemias or lymphomas.
Leukaemias or lymphomas aimed at by the present invention are e.g. AML, CML, ALL, CLL or non-Hodgkin lymphoma as defined above.

The figures show:

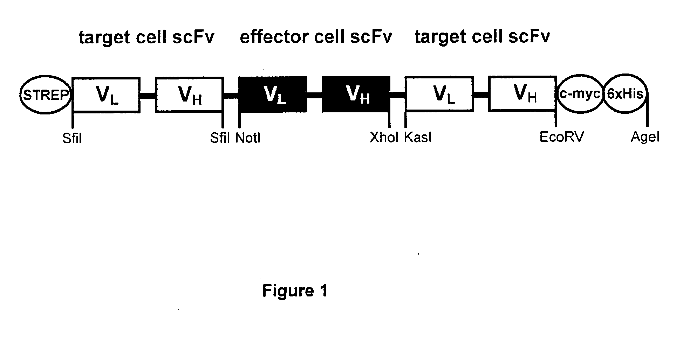

Figure 1. Construction of a recombinant tandem single-chain Fv triple-body (or triabody; sctb). The two distal scFvs are specific for the tumour antigens and the central scFv is directed against an activating trigger molecule on the effector cells. CMV, *cytomegalovirus immediate early promotor*, Igκ, secretion leader sequence from the murine Ig kappa L chain; V_{L},V_{H}, cDNA sequences coding for the V regions of Ig L-or H-chains; Strep, c-myc, 6 x His, cDNA coding for a strep, c-myc or a hexahistidine tag.

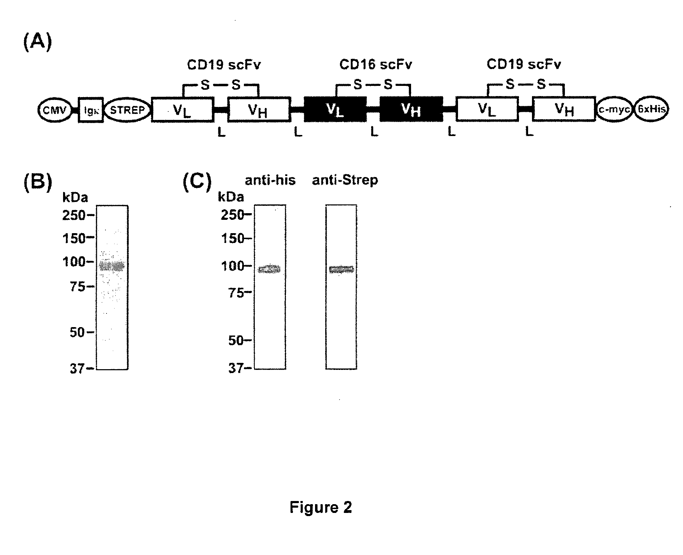

Figure 2. Construction and expression of the sctb disulphide-stabilized (ds) [19x16x19]. (A) Design of the sctb ds[19x16x19]. The two distal scFvs are specific for the tumour antigen CD19 and the central scFv is directed against the activating trigger molecule CD16 on NK cells and macrophages. CMV, *cytomegalovirus immediate early promotor;* Igκ, secretion leader sequence from the murine Ig kappa L chain; V_{L},V_{H}, cDNA sequences coding for the V regions of Ig L-or H-chains; L, cDNA coding for a 20 amino acid flexible linker, e.g. (Gly₄Ser)₄; Strep, c-myc, 6 x His, cDNA coding for a strep, c-myc or a hexahistidine tag; S-S, stabilising disulphide bond. Integrity and purity of the sctb ds[19x16x19] after affinity chromatography with Ni-NTA agarose beads as evaluated by reducing SDS-PAGE and staining with Coomassie blue (B) and Western Blot analysis using anti-his or anti-Strep antibodies for detection, respectively (C).

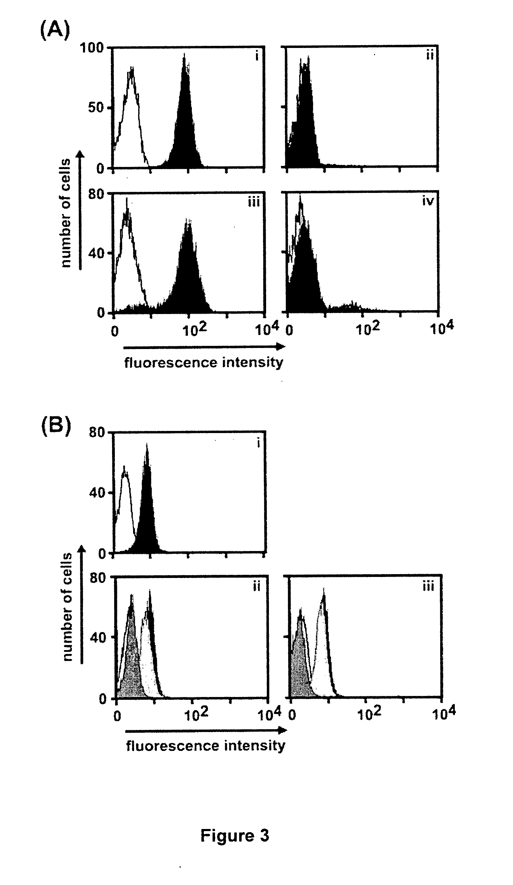

Figure 3. Specific and simultaneous antigen binding of the sctb ds[19x16x19]. (A) FACS analysis of specific binding of the sctb ds[19x16x19] to CD19-positive SEM cells (i), CD19-negative CEM cells (ii), CD16-transfected CHO cells (iii), and untransfected CHO cells (iv). (white peak: signal from isotype control; black peak: signal from sctb ds[19x16x19]). (B) Simultaneous binding to CD19 and CD16. SEM cells were incubated with the sctb ds[19x16x19] and simultaneous binding was revealed by addition of a fusion protein consisting of the extracellular domain of CD16 and GFP (CD16ex-GFP) (i). The fluorescence signal produced by CD16ex-GFP was blocked by pre-incubation of SEM cells with of a 50-fold molar excess of the CD19 mAb 4G7 (ii) or by pre-incubating the CD16ex-GFP fusion protein with the CD16 mAb 3G8 (iii). No blocking occurred by addition of a non-relevant IgG1 isotype. (white peak: signal from control sctb; black peak: signal from sctb ds[19x16x19]; dark grey peak: signal obtained after addition of parental mAb; light grey peak: signal after addition of IgG1 isotype).

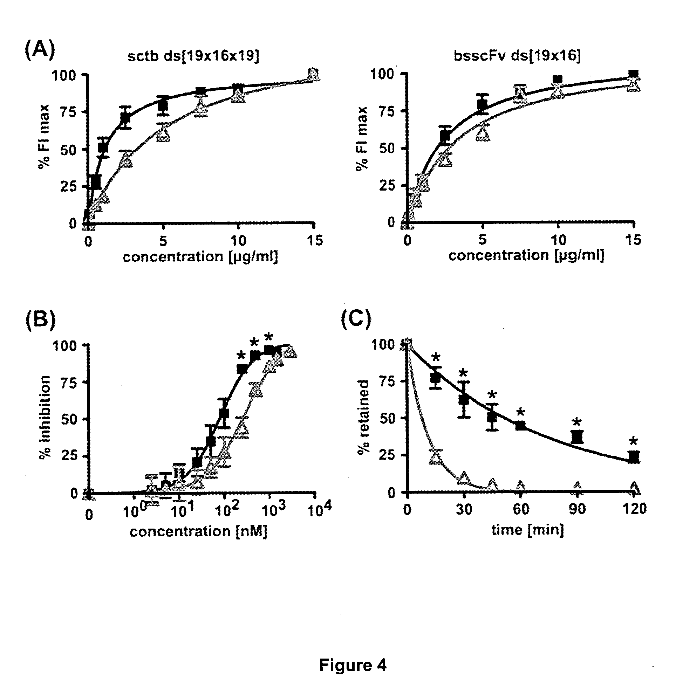

Figure 4. Comparision of the binding activities of the sctb ds[19x16x19] and the bi-specific (bs) scFv ds[19x16]. (A) Equilibrium binding curves for the sctb ds[19x16x19] (left) and the bsscFv ds[19x16] (right) obtained by flow cytometry. Increasing concentrations of the sctb and the bsscFv were added to CD19-positive SEM (filled black square) or to CD16-transfected CHO cells (open grey triangle) and detected with an anti-His antibody. K_{D} values were calculated by the Langmuir/Scatchard algorithm. (B) Concentration-dependent competitive inhibition of the binding of the CD19 mAb 4G7 to SEM cells. Decrease of binding of mAb 4G7 with increasing concentrations of the inhibitors sctb ds[19x16x19] (filled black square) or bsscFv ds[19x16] (open grey triangle) was monitored by flow cytometry. For each concentration of the inhibitors the resulting percentage of inhibition was calculated. (C) Cell surface retention *in vitro.* SEM cells were decorated with the sctb ds[19x16x19] (filled black square) or the bsscFv ds[19x16] (open grey triangle), respectively, and incubated at 37°C. After washing away excess molecules, aliquots were removed and analysed for molecules retained on the cell surface by flow cytometry at the indicated time points. *Statistically significant difference between the sctb ds[19x16x19] and the bsscFv ds[19x16].

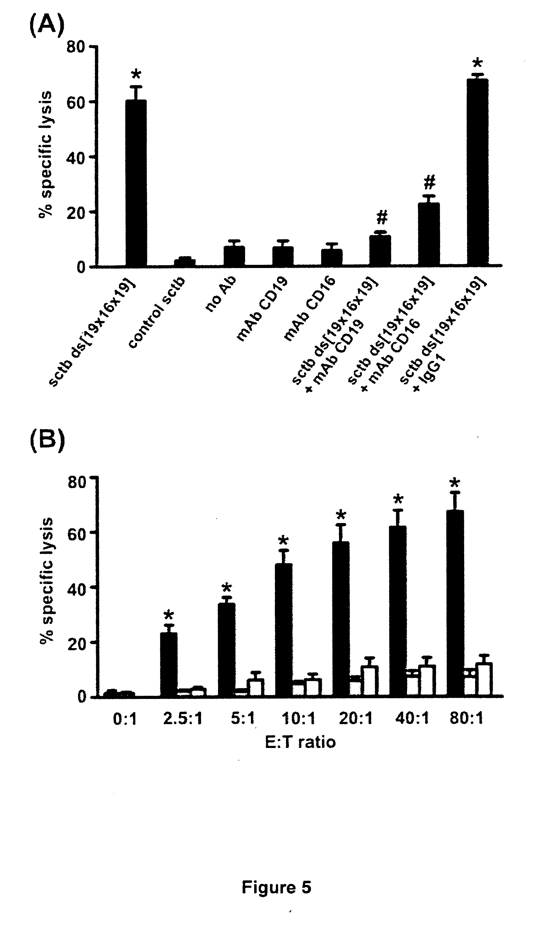

Figure 5. The sctb ds[19x16x19] mediates potent lysis of CD19-positive SEM target cells by freshly isolated MNCs as effector cells. (A) Induction of ADCC is CD19-specific and CD16-dependent. The sctb ds[19x16x19] induced specific lysis at the concentration of 1 nM at an effector to target (E:T) cell ratio of 40:1. Neither the non-relevant control sctb, nor the parental mAbs 4G7 (CD19) and 3G8 (CD16) alone were able to induce significant lysis at equimolar concentration. ADCC was competitively inhibited by addition of a 125-fold molar excess of the parental mAbs 4G7 or 3G8, but not by a corresponding excess of an IgG1 isotype. (B) At the concentration of 1 nM the sctb ds[19x16x19] induced significant ADCC over a broad range of E:T ratios and the extent of specific lysis increased with increasing E:T ratios (black bars: sctb ds[19x16x19]; grey bars: control sctb, white bars: no antibody). Data are presented as mean percentage lysis ± standard error of the mean (SEM) obtained with isolated MNCs from at least three different healthy donors. *Statistically significant differences in ADCC compared to the control without added antibody. #Statistically significant differences in ADCC compared to the sctb ds[19x16x19] added alone.

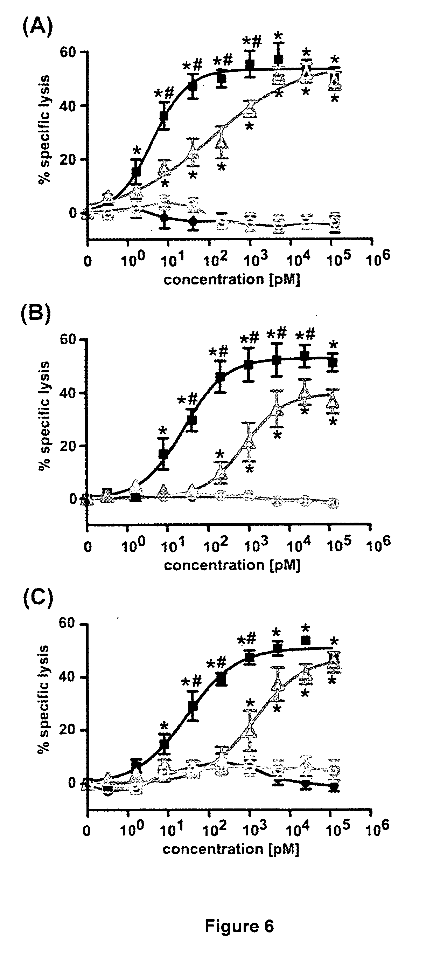

Figure 6. Dose dependent induction of ADCC of different tumor cell lines by the sctb ds[19x16x19] and the bsscFv ds[19x16]. The CD19-positive tumour cell lines SEM (A), BV-173 (B) and ARH-77 (C) were used as targets to compare efficacy of both the sctb and the bsscFv at a constant E:T cell ratio of 40:1. The sctb ds[19x16x19] (filled black square) and the bsscFv ds[19x16] (open grey triangle) triggered ADCC in a dose-dependent manner. Neither the non relevant control sctb (filled black circle) nor the non relevant control bsscFv (open grey circle) induced significant killing. Data points represent mean percentage of lysis ± SEM obtained with isolated MNCs from at least six different healthy donors. *Statistically significant differences in ADCC compared to the control without added antibody. #Statistically significant differences between killing induced by sctb ds[19x16x19] and bsscFv ds[19x16].

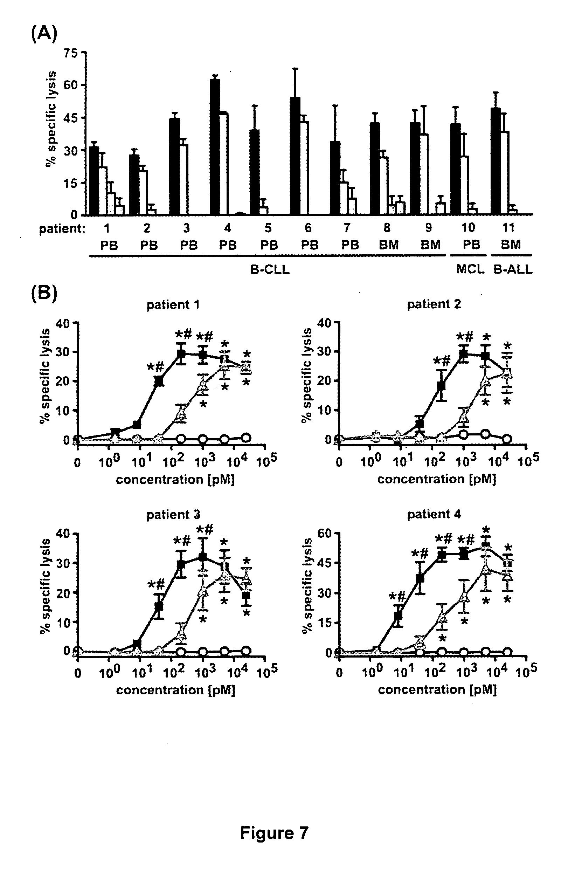

Figure 7. Potent lysis of CD19-positive primary lymphoma cells and leukaemia blasts by the sctb ds[19x16x19] and the bsscFv ds[19x16]. (A) Malignant cells from peripheral blood (PB) or bone marrow (BM) from nine B-CLL patients, and from one MCL and one B-ALL patient, were lysed by the sctb ds[19x16x19] (black bars) and the bsscFv ds[19x16] (white bars) at the concentration of 1 nM, using MNCs from a healthy donor at an E:T ratio of 40:1. No lysis was induced by a non relevant sctb (dark grey bars) or by MNCs alone (light grey bars). Data points are presented as mean values from triplicate determinations, error bars represent SEM. (B) Dose-response curves using cells from B-CLL patients 1 - 4 as targets at an E:T ratio of 40:1. The sctb ds[19x16x19] (filled black square) induced lysis with 10- to 20-fold lower EC₅₀ values than the bsscFv ds[19x16] (open grey triangle). No significant lysis was observed with a non-relevant control sctb (open circle). Data points represent mean percentage of lysis ± SEM obtained with isolated MNCs from at least three different healthy donors. *Statistically significant differences in ADCC compared to the control without added antibody. #Statistically significant differences between killing induced by the sctb ds[19x16x19] and the bsscFv ds[19x16].

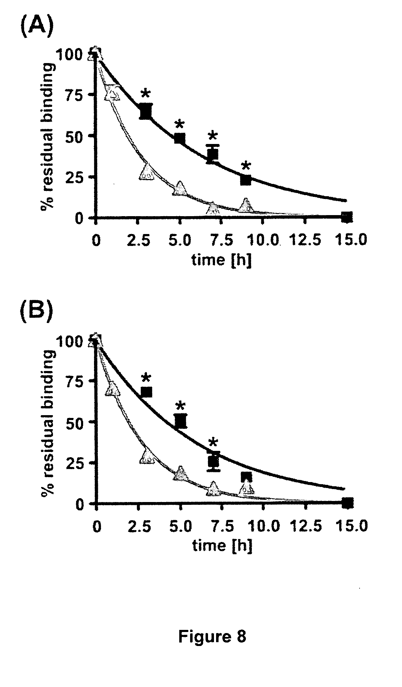

Figure 8. *In vivo* plasma retention times. The sctb ds[19x16x19] (filled black square) and the bsscFv ds[19x16] (open grey triangle) were separately injected i.v. into NOD-SCID mice. The mice were bled at several time points and serum was analysed for the presence of immunoreactive sctb and bsscFv by flow cytometry using CD19-positive SEM (A) and CD16-transfected CHO cells (B). *Statistically significant differences between residual binding of the sctb ds[19x16x19] and the bsscFv ds[19x16].

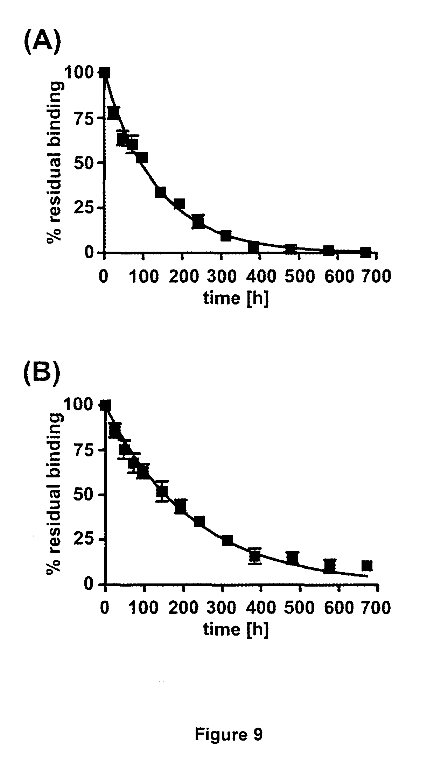

Figure 9. Serum stability *in vitro* at 37°C. The sctb ds[19x16x19] (filled black square) was incubated for several periods of time in human serum at 37°C. Residual binding capacities of the CD19 and CD16 binding moieties were determined by flow cytometry. (A) Residual binding to CD19-positive SEM cells. (B) Residual binding to CD16-transfected CHO cells.

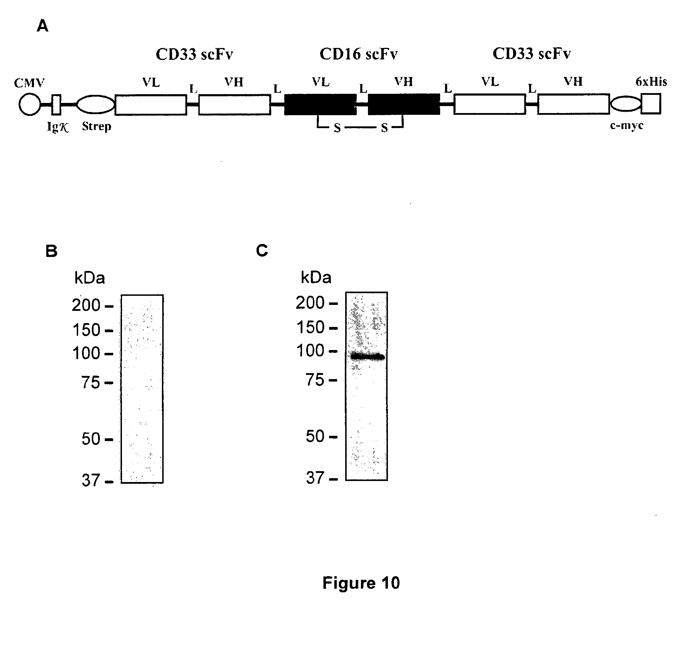

Figure 10. Construction and expression of the sctb [33xds16x33]. (A) Design of the sctb [33xds16x33]. The two distal scFvs are specific for the myeloid tumour antigen CD33 and the central scFv is directed against the activating trigger molecule CD16 on NK cells and macrophages. CMV, *cytomegalovirus immediate early promotor;* Igκ, secretion leader sequence from the murine Ig kappa L chain; V_{L},V_{H}, cDNA sequences coding for the V regions of Ig L-or H-chains; L, cDNA coding for a 20 amino acid flexible linker (Gly₄Ser)₄; Strep, c-myc, 6 x His, cDNA coding for a strep, c-myc or a hexahistidine tag; S-S, stabilising disulphide bond. Integrity and purity of the sctb [33xds16x33] after affinity chromatography with Ni-NTA agarose beads as evaluated by reducing SDS-PAGE and staining with Coomassie blue (B) and Western Blot analysis using an anti-his antibody for detection (C).

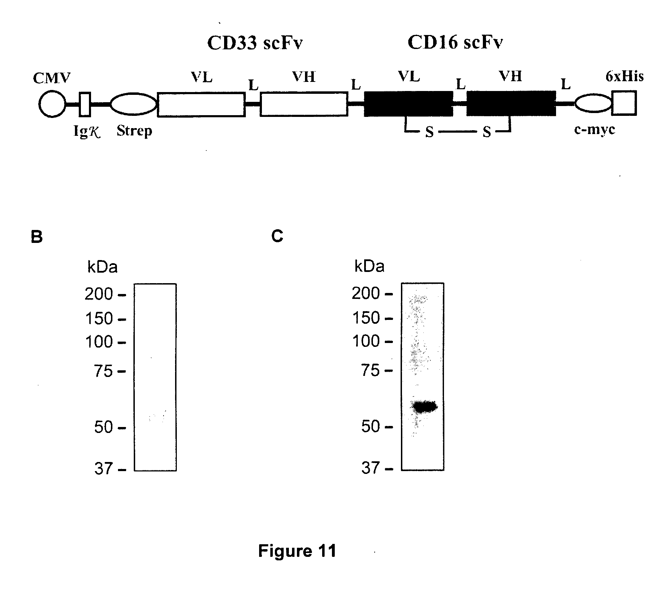

Figure 11. Construction and expression of the bsscFv [33xds16]. (A) Design of the bsscFv [33xds16]. The N-terminal scFv is specific for the tumour antigen CD33 and the C-terminal scFv is directed against the activating trigger molecule CD16 on NK-cells and macrophages. CMV, cytomegalo immediate early promoter, Igκ, secretion leader sequence from the murine Ig kappa L chain; V_{L}, V_{H}, cDNA sequences coding for the V regions of Ig L- or H-chains; L, cDNA coding for a 20 amino acid flexible linker (Gly₄Ser)₄; Strep, c-myc, 6xHis, cDNA coding for a strep, c-myc or a hexahistidine tag; S-S, stabilising disulphide bond. Integrity and purity of the bsscFv [33xds16] after affinity chromatography with Ni-NTA agarose beads as evaluated by reducing SDS-PAGE and stained with Coomassie blue (B) and Western Blot analysis using an anti-his antibody for detection(C).

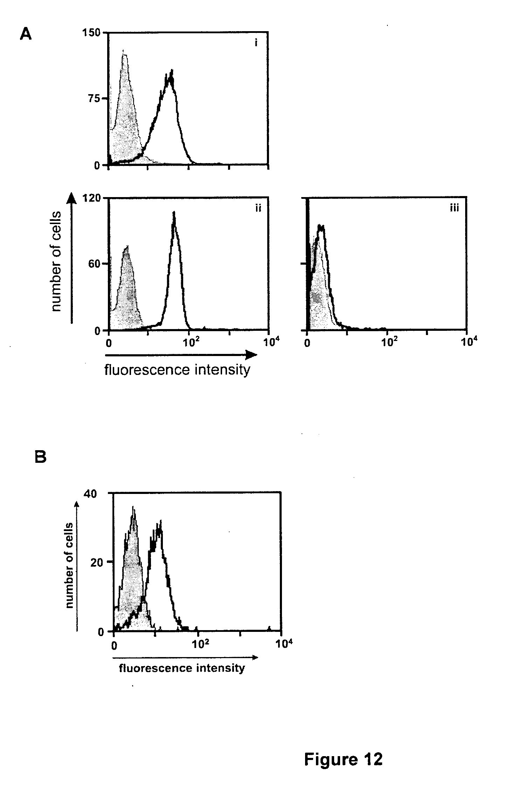

Figure 12. The sctb [33xds16x33] binds specifically and simultaneously to the respective antigens. (A) Specific binding of the sctb [33xds16x33] to CD33-positive HL-60 cells (i), CD16-transfected CHO cells (ii) and CD33 and CD16 antigen negative SEM cells (iii) was examined via fluorescence analysis. (grey peak: signal from control sctb; white peak: signal from sctb [33xds16x33]. (B) Simultaneous binding to CD16 and CD33. CD16-transfected CHO cells were incubated with the sctb [33xds16x33] and simultaneous binding was revealed by addition of a fusion protein consisting of the extracellular domain of CD33 and DsRed (CD33ex-DsRed) (grey peak: signal from control sctb; white peak: signal from sctb [33xds16x33]).

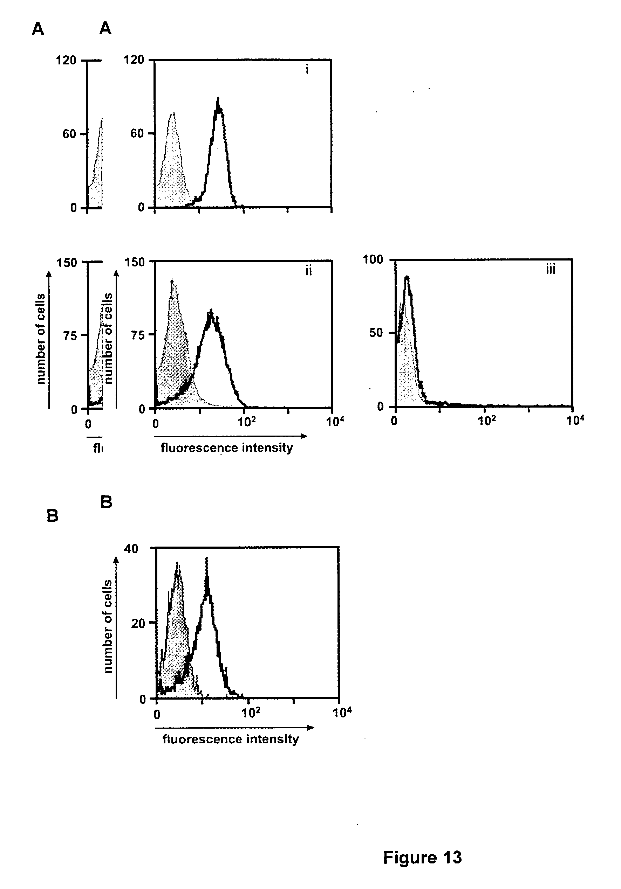

Figure13. The control protein bsscFv [33xds16] binds specifically and simultaneously to the respective target antigens. (A) Specific binding of the bsscFv [33xds16] to CD33-positive HL-60 cells (i), CD16-transfected CHO cells (ii) and CD33 and CD16 antigen negative SEM cells (iii) was examined via fluorescence analysis. (grey peak: signal from control bsscFv; white peak: signal from sctb [33xds16]. (B) Simultaneous binding to CD16 and CD33. CD16-transfected CHO cells were incubated with the bsscFv [33xds16] and simultaneous binding was revealed by addition of a fusion protein consisting of the extracellular domain of CD33 and DsRed (CD33ex-DsRed) (grey peak: signal from control bsscFv; white peak: signal from bsscFv [33xds16]).

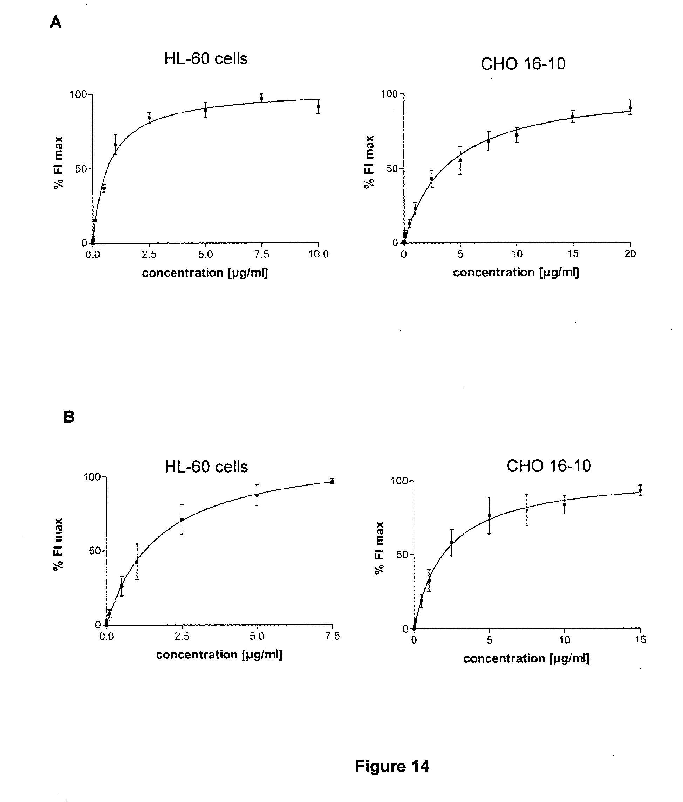

Figure 14. Equilibrium binding curves of the sctb [33xds16x33] and the bsscFv [33xds16]. Equilibrium binding curves were obtained via flow cytometry. CD33-positive HL-60 cells (left) or CD16-transfected CHO cells (CHO 16-10) (right) were incubated with increasing concentrations of the antibody fragments and detected with an anti-His antibody. The K_{D}-values were calculated by the Langmuir/Scatchard algorithm (Benedict et al, 1997). (A) sctb [33xds16x33], (B) bsscFv [33xds16]

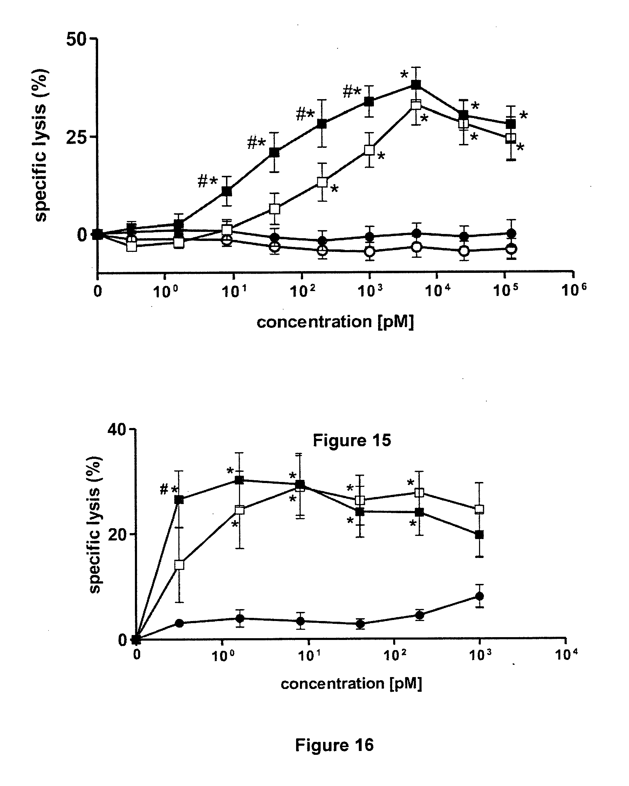

Figure 15. Dose dependent induction of ADCC by the sctb [33xds16x33] and the bsscFv [33xds16]. The CD33-positive tumour cell line HL-60 was used as target to compare efficacy of both the sctb and the bsscFv at a constant E:T cell ratio of 40:1. The sctb [33xds16x33] (filled square) and the bsscFv [33xds16] (open square) triggered ADCC in a dose-dependent manner. Neither the non relevant control sctb (filled circle) nor the non relevant control bsscFv (open circle) induced significant killing. Data points represent mean percentage of lysis ± SEM obtained with isolated MNCs from eight different healthy donors. *Statistically significant differences in ADCC compared to the respective isotype antibody. #Statistically significant differences between killing induced by sctb [33xds16x33] and bsscFv [33xds16].

### Example 1: Construction of a tandem scFv triple-body (triabody)

A single-chain Fv triple-body (triabody; sctb) consists of one single polypeptide chain with three scFvs connected in tandem (Fig. 1). Preferably the distal scFvs can be specific for any tumour antigen, and the central scFv for any trigger molecule on effector cells, such as CD16 (FcγRIII) on NK cells and macrophages. Other arrangements are also possible such that one of the terminal scFvs can be directed against an antigen on the effector cells, while the two other scFvs are specific for an antigen expressed on the tumour cells.

To generate the format of a recombinant tandem single chain Fv triple-body (sctb) three scFvs, each comprising a variable light and a variable heavy chain connected via a 20 aa linker (Krebber et al., 1997), have to be fused together with a flexible linker (e.g. 20 aa (G₄S)₄). For the construction of tandem single chain Fv triple-bodies (triabodies) a multiple cloning site (MCS) has to be ligated into a suitable expression vector, e.g. into the eukaryotic expression vector pSecTag2HygroC (Invitrogen, Karlsruhe, Germany) using appropriate restriction sites. This multiple cloning site and the resulting vector have to provide the restriction sites for the insertion of three scFvs, the linker sequences, tags for purification and detection (e.g. 6xHis, Strep; c-myc), a secretion leader (e.g. Ig kappa chain leader sequence) as well as elements necessary for expression in the chosen system, for eukaryotic expression e.g. a cytomegalus virus (CMV) promotor and an poly-adenylation signal. A resistance cassette (e.g. hygromycin C) will be helpful for the generation of a stably transformed production cell line. The above vector can be genetically engineered in that a first scFv can be inserted as a Sfil cassette, a second scFv as a NotI/XhoI cassette and a third scFv as a Kasl/EcoRV cassette. The use of other restriction enzymes is also possible. Each scFv can then be replaced against another scFv, which provides a system for a rapid recombination of scFvs of different specificities (Figure 1). If necessary, disulfide stabilized scFv variants should be used to achieve high serum stability at 37°C.
In this format two scFvs directed against a tumour antigen, e.g. CD19 can be combined with a scFv directed against a effector antigen e.g. CD16, generating sctb [19x16x19]. If necessary, respective scFvs can be amplified using Polymerase Chain Reaction (PCR), thereby introducing the restriction sites needed for cloning them into the vector using standard cloning proceedures (Sambrook & Russel, 2001).

### Example 2: Expression and purification of a tandem scFv triple-body (triabody)

The respective tandem scFv triple-body can be expressed in eukaryotic cells e.g. 293T after transient transfection using the calcium phosphate technique including chloroquine (Sambrook & Russel, 2001). Preferably, a production cell line is generated by stable transfection with the linearized (e.g. enzyme FspI for derivatives of the vector pSecTag2HygroC) vector coding for this tandem scFv triple-body. Positive clones can be selected by e.g. 200µg/ml hygromycin C (Carl Roth, Karlsruhe, Germany) and can subsequently be singularised. For expression, monoclonal stably transfected eukaryotic cells can be cultured under permanent selection in a mini-PERM bioreactor (Greiner Bio-One, Frickenhausen, Germany) with a dialysis membrane of 12.5 kDa in accordance with the manufacturer's instructions. The medium containing recombinant protein has usually to be collected 4 times in a period of two weeks and dialysed at 4°C against a 4000 fold excess of buffer containing 50mM NaH₂PO₄, 300mM NaCl, 10mM Imidazole, pH 8.0. The recombinant tagged proteins can be purified e.g. by affinity chromatography with nickel-nitrilotrioacetic acid (Ni-NTA) agarose beads (Qiagen) and finally dialysed against PBS.

Eluted protein (e.g. sctb[19x16x19]) can be analyzed by reducing Sodium dodecyl sulphate polyacrylamide gel electrophoresis (SDS-PAGE) using standard procedures (Laemmli,1970). Gels are stained with Coomassie brilliant blue R250 (Sigma-Aldrich, Taufkirchen, Germany) will show a protein of approximate Mᵣ = 80-95 kDa. In Western blot analyses, tandem scFv triple-bodies (triabodies) can be detected using antibodies directed against respective tags, e.g. with an unconjugated penta-His antibody (Qiagen, Hilden, Germany) and secondary horseradish peroxidase (HRP) coupled sheep anti-mouse IgG (Dianova, Hamburg, Germany) antibody, or with HRP conjugated Strep Tag antibody (IBA, Goettingen, Germany). Western Blots are developed using enhanced chemiluminescence reagents (Amersham Pharmacia Biotech, Freiburg, Germany).

This purified sctb can e.g. be used for the following experiments:

### Example 3: Determination of antibody equilibrium constants (K_{D}-values)

To asses whether incorporation of a second target cell scFv results in a gain of affinity/avidity, antibody equlilibrium constants (K_{D} values; antibody concentration, at which half maximal binding will be achieved) can be determined. These can be measured by flow cytometry as described previously (Benedict et al, 1997; Bruenke et al., 2004). Immunofluorescence staining should be performed on a FACSCalibur instrument using CellQuest software (Becton Dickinson, Heidelberg, Germany). Briefly, tumour antigen positve cells, e.g. CD19 positive SEM cells (Greil et al., 1994) are incubated with increasing concentrations of recombinant antibody fragments on ice. After washing, antibody fragments can be detected using appropriate antibodies e.g. penta-His antibody and phycoerythrin (PE)-conjugated goat anti-mouse IgG (DAKO Diagnostica GmbH, Hamburg, Germany). 1 x 10⁴ events are collected for each sample and whole cells are analysed using appropriate scatter gates to exclude cellular debris and aggregates. Experiments are repeated 6 times and values and graphical analyses are generated with GraphPad Software (Graph Pad software Inc., San Diego, CA, USA) using nonlinear regression curve-fit after normalising maximal fluorescence intensity to 100 %. Group data should be reported as means ± standard error of the mean (SEM). Differences between K_{D} values are analyzed using unpaired Student's t-test. P-values < 0.05 are considered significant. Likewise, the affinity to the effector cell antigen, e.g. CD16, can be measured. In these experiments the tandem scFv triple-body will be compared to a monovalent conventional bsscFv (Bruenke et al., 2004), consisting of the same scFvs used for construction of the tandem scFv triple-body, e.g. one CD19- and one CD16-directed scFv, derived from the same hybridomas. If both tumour cell directed binding moieties are functional and contribute to antigen binding, the tandem scFv triple-body should have an higher affinity/avidity to the tumour cells as compared to its monovalent counterpart and should exhibit a K_{D} value in low nanomolar ranges. Preferably, the affinity to the effector cell antigen, e.g. CD16, should not be altered by the additional N- or C-terminal fusion of a further scFv. For the effector cell antigen the tandem scFv triple-body should show a K_{D} value that is 4 to 6 times higher as compared to the tumour cell antigen. Hence, the affinity/avidity of this molecule will expectedly be shifted towards the leukaemia cells, as desired.

### Example 4: Effectiveness of the tandem scFv triple-body (triabody) as compared to corresponding bispecific antibody bsscFv.

The tandem scFv triple-body can be compared side-by-side to the bsscFv with respect to induction of antibody dependent cytotoxicity (ADCC) by effecor cells, e.g. mononuclear cells (MNCs). ADCC assays with MNCs of healthy donors and tumour derived antigen-positive cell lines will be performed in triplicate by a 3-hour ⁵¹Chr release assay as described (Elsasser et al. 1996). At a constant effector to target (E:T) ratio of 40:1 the antibody derivatives are added to the samples in several eqimolar 5-fold serial dilutions and the EC₅₀ values (concentration of antibody derivative that yields 50% of maximal obtained tumour cell lysis) are calculated after substraction of background lysis with Graph-Pad Prism Software by nonlinear regression curve fit. Group data are reported as means ± SEM of at least 4 different experiments. Differences between groups are analyzed using paired Student's t-test. P-values < 0.05 will be considered significant.
The tandem scFv triple-body should be capable to induce ADCC and lyse at least three different tumour derived cell lines e.g. SEM in a dose dependent manner with EC₅₀ values in picomolar range. Under identical conditions also the bsscFv antibody should mediate cell lysis, but efficacy is supposed to be considerably lower. The calculated EC₅₀ values are expected to be significantly higher and could be 30 to 40 times higher.
If corresponding data are experimentally confirmed, this will clearly illustrate that under identical experimental conditions the tandem scFv triple-body would exhibit a superior lytic activity compared to the bsscFv.

### Example 5: Material and Methods

### Cell lines and hybridomas

Chinese hamster ovary (CHO) cells, stably transfected with a human CD16A cDNA expression vector, were from Dr. J. van de Winkel (University Medical Centre, Utrecht, The Netherlands). The 4G7 hybridoma (CD19, mlgG1; Meeker et al, 1984) was from Dr. R. Levy (Stanford University, Palo Alto, CA, USA). The hybridoma 3G8 (FcγRIII, CD16, mlgG1; Fleit et al, 1982) was from the American Type Cell Culture Collection (ATCC, Manassas, VA, USA). CHO cells, the hybridomas, and the B-precursor ALL derived cell line SEM (Greil et al., 1994), the EBV-transformed B-lymphoblastioid cell line ARH-77 (ATCC), the T-ALL derived cell line CEM (ATCC), and the pre-B phenotypic cell line BV-173 (German Collection of Microorganisms and Cell Cultures, DSMZ, Braunschweig, Germany) and the AML-derived cell line HL-60 (ATCC) were cultured in Roswell Park Memorial Institute (RPMI) 1640 Glutamax-I medium (Invitrogen, Karlsruhe, Germany) containing 10% foetal calf serum (FCS; Invitrogen), 100 units/ml penicillin (Invitrogen) and 100 µg/ml streptomycin (Invitrogen). Human embryonal kidney 293T cells (ATCC) were maintained in Dulbecco's Modified Eagle's Medium (DMEM) Glutamax-I medium (Invitrogen) supplemented with 10% FCS, penicillin and streptomycin at 100 units/ml and 100 µg/ml, respectively.

### Construction of recombinant tandem scFv triple-bodies (scFv triple-bodies or triabodies; sctb) and fusion proteins

*Escherichia coli* strain XL-1 blue (Stratagene, Amsterdam, The Netherlands) was used as host for the amplification of the plasmids and cloning. To generate the expression vector for the sctb ds[19x16x19], the cDNA coding for the disulphide stabilized (ds) variant of the CD19 scFv was excised from the vector pSecTag2HygroC-Strep-dsCD19 4G7xdsCD16 (Bruenke et al, 2005) and cloned as an Sfil cassette into a derivative of the vector pSecTag2HygroC (Invitrogen), generating vector pSecTag2HygroC-Strep-ds19. To construct pSecTag2HygroC-Strep-ds19xds16xds19, the sequences encoding scFv dsCD16 and scFv dsCD19 were amplified by PCR from pSecTag2HygroC-Step-dsCD19 4G7xdsCD16 and ligated into pSecTag2HygroC-Strep-ds19, using NotI/XhoI and Xhol/Agel restriction sites, respectively. The expression vector pSecTag2HygroC-Strep-7xds16x7 was constructed by exchange of the coding sequences for the CD19 scFv with those encoding the CD7 scFv TH69 (Peipp et al., 2002) using equivalent restriction sites. The expression vector for the control bsscFv [7xds16], pSecTag2HygroC-Strep 7xds16, was generated by replacing the sequence coding for the CD19 scFv in pSecTag2HygroC-Strep ds CD19 4G7xdsCD16 with that coding for the scFv TH69 as an Sfil fragment.
To construct the expression vector for the sctb [33xds16x33], the coding sequences for the CD33 scFv was excised from the vector pet27b(+)-Strep-His-CD33-ETA-KDEL (Schwemmlein et al., 2006) and cloned as Sfi cassette into the vector pSecTag2HygroCStrep-ds19xds16xds19, replacing the coding sequences for the N-terminal CD19 scFv and generating pSecTag2HygroCStrep 33xds16xds19. To generate the vector pSecTag2HygroC-Strep 33xds16x33, the sequence coding for the CD33 scFv was amplified by PCR from pet27b(+)-Strep-His-CD33-ETA-KDEL and ligated into pSecTag2HygroCStrep 33xds16xds19, using Xhol/EcoRV restriction sites, replacing the coding sequences for the C-terminal CD19 scFv.
The expression vector for the control bsscFv [33xds16], pSecTag2HygroC-Strep 33xds16 was generated by replacing the sequence coding for the dsCD19 scFv in pSecTag2HygroC-Strep dsCD19 4G7xdsCD16 with that coding for the CD33 scFv as an Sfil fragment.
To construct the expression vector for the CD33ex-DsRed fusion protein, the cDNA coding for the extracellular domain of CD33 was amplified by PCR from the vector pSecTag-ChCD33-Fc (Schwemmlein et al., 2006) and ligated as an Sfil cassette into the vector pSEC-Tag Hygro-DsRed (Peipp et al., 2003).
To confirm correct construction, the final constructs were sequenced (Sambrook and Russel, 2001) on an Applied Biosystems automated DNA sequencer (ABI Prism 310 Genetic Analyzer; Perkin-Elmer, Ueberlingen, Germany).

### Expression and purification of recombinant antibody fragments and fusion proteins

For expression of the recombinant single-chain Fv triple-bodies sctb ds[19x16x19], sctb [33xds16x33] and sctb [7xds16x7], 293T cells were transfected with the respective expression vectors using the calcium phosphate technique including chloroquine (Sambrook and Russel, 2001). Stable production cell lines for each construct were obtained after transfection with the respective vectors linearized with the restriction enzyme Fspl. Positive clones were selected in the presence of 200 µg/ml Hygromycin B (Roth, Karlsruhe, Germany), and single cell clones were isolated by limiting dilution. Supernatants were analysed for the presence of antibody fragments by flow cytometry. For high-grade expression, cell clones were cultured under permanent selection in a mini-PERM bioreactor (Greiner Bio-One, Frickenhausen, Germany) using a membrane with a pore size corresponding to proteins ≤ 12.5 kDa, in accordance with the manufacturer's instructions, and the medium containing the recombinant protein was collected 4 times over a period of 2 weeks. The recombinant His-tagged proteins were purified by affinity chromatography with nickel-nitrilotriacetic acid (Ni-NTA) agarose beads (Qiagen) as described (Bruenke et al, 2005) and finally dialysed against phosphate-buffered saline (PBS).

The corresponding bispecific scFv fragments used as controls, bsscFv ds[19x16], bsscFv [33xds16] and bsscFv [7xds16], were produced and purified in parallel under identical conditions. Fusion proteins with the green fluorescent protein (GFP) and the red fluorescent protein DsRed were expressed in 293T cells and purified as described previously (Bruenke et al, 2004).

### SDS-PAGE and Western blot analysis

SDS-PAGE was performed by standard procedures (Sambrook and Russel, 2001). In Western blot experiments, the recombinant proteins were detected either with a horse radish peroxidase (HRP)-conjugated antibody specific for the Strep Tag (IBA, Goettingen, Germany), or with an unconjugated penta-His antibody (Qiagen, Hilden, Germany), and a secondary HRP-coupled goat anti-mouse IgG antibody (Dianova, Hamburg, Germany). Western Blots were developed using enhanced chemiluminescence reagents (Amersham Pharmacia Biotech, Freiburg, Germany).

### Flow cytometric analysis

Immunofluorescence analysis was performed on a FACSCalibur instrument using CellQuest software (Becton Dickinson, Heidelberg, Germany) as described (Bruenke et al, 2004). Briefly, 1 x 10⁴ events were collected for each sample, and whole cells were analysed using appropriate scatter gates to exclude cellular debris and aggregates. The recombinant antibody fragments were detected using a penta-His antibody and a phycoerythrin (PE)-conjugated goat anti-mouse IgG antibody (DAKO Diagnostica GmbH, Hamburg, Germany), unless otherwise stated.

### Determination of equilibrium constants (K_{D})

K_{D} values were determined using methods based on flow cytometry, as described (Bruenke et al, 2004; Benedict et al, 1997). The highest mean fluorescence value was set to 100% and all data points were normalized. The experiments were repeated 4 to 7 times and mean values are reported. The K_{D} values were calculated using a nonlinear regression curve fit.

### Cell surface retention assay

Cell surface retention assays were performed by published procedures, under conditions preventing the internalisation of antigens (Adams, et al, 1998). Briefly, 4 x 10⁶ CD19-positive SEM or CD16-transfected CHO cells were incubated for 1 h on ice, with 10 µg/ml of either the sctb ds[19x16x19] or the bsscFv ds[19x16]. Cells were washed twice with 12 ml of cold FACS buffer (0.154 M sodium chloride, 10 mM sodium phosphate, 1% bovine serum albumin, 0.1% sodium azide, pH 7.2) and then collected by centrifugation. The cells were resuspended in 4 ml FACS buffer and incubated at 37°C. At several time points the cells were washed again to remove dissociated molecules and resuspended in FACS buffer. Aliquots of 0.5 x 10⁶ cells were removed, placed on ice for 5 min, and washed again. Molecules retained on the cell surface were then detected by FACS, using an Alexa Fluor 555 conjugated anti penta-His antibody (Qiagen). All data points were normalized to the time point to = 100 %. The experiment was performed 3 times and mean values are reported. Half-life values for cell surface retention were then obtained by fitting the data to a single-phase exponential decay.

### Competitive inhibition assay

SEM cells were incubated on ice with the monoclonal antibody (mAb) 4G7 at a subsaturating concentration of 0.5 µg/ml either alone or in the presence of varying concentrations of either the sctb ds[19x16x19] or the bsscFv ds[19x16] as inhibitors. After washing, the mAb 4G7 was detected on the cells with a PE-labeled goat anti-mouse IgG antibody (DAKO Diagnostica) by flow cytometry. Relative inhibition of binding of mAb 4G7 was calculated as follows: % inhibition = [(% FI without inhibitor - % FI with inhibitor) / (% FI without inhibitor)] x 100 (FI, fluorescence intensity). The experiment was performed 5 times and mean values are reported. IC₅₀ values (concentration of inhibitor producing 50% inhibition of binding of mAb 4G7) were calculated using a sigmoidal dose response curve fit.

### Isolation of MNCs and malignant leukaemia and lymphoma cells from human donors

Citrate buffered or heparinized peripheral blood (PB) and bone marrow (BM) samples, from both healthy volunteers and leukaemia or lymphoma patients, were obtained after receiving informed consent, and with the approval of the Ethics Committee of the University of Erlangen-Nuremberg. MNCs were enriched by Lymphoflot (Biotest, Dreieich, Germany) Ficoll density centrifugation in Leukosep tubes (Greiner, Frickenhausen, Germany) according to manufacturers' instructions and suspended in RPMI 1640 Glutamax-I medium containing 10% FCS and penicillin and streptomycin at 100 units/ml and 100 µg/ml, respectively. Viability was verified by Trypan blue exclusion and exceeded 95%. Patient derived samples were analysed for the expression of CD19 by staining with a PE-conjugated CD19 antibody (BD Biosciences, Heidelberg, Germany). The expression of CD33 on AML blasts was assessed with a monoclonal CD33 mouse antibody (Dako Cytomation, Glostrup, Denmark) that was detected with an polyclonal PE-conjugated goat anti-mouse IgG antibody (Dako Cytomation, Glostrup, Denmark) and flow cytometry.

### ADCC reactions

ADCC assays, using MNCs from healthy donors as effector cells, were performed in triplicate using a 3 hour ⁵¹Cr release assay as described (Elsasser et al, 1996). For blocking experiments, the parental antibodies 4G7 and 3G8 were added at a 125-fold molar excess. Dose response curves were recorded using several equimolar 5-fold serial dilutions of the respective antibody fragments at a constant effector to target (E:T) cell ratio of 40:1. Background lysis induced by MNCs alone was subtracted from each data point, and EC₅₀ values (concentration of an antibody fragment producing 50% of maximum specific lysis) were calculated by using a sigmoidal dose response curve fit. The experiments were repeated 4 to 7 times and mean values are reported.

### Measurement of in vitro serum stability

The sctb ds[19x16x19] was incubated in human serum at sub-saturating concentrations of 2.5 µg/ml in a total volume of 220 µl at 37°C. Residual binding activity was determined at various time points by flow cytometry. The time point to was set to 100%, and all data were normalized to this value. The experiment was performed 7 times and mean values are reported. The half-life values for each of the binding sites were calculated from a one-phase exponential decay curve fit.

### Determination of in vivo plasma half-life in mice

Animal care and all experiments were performed in accordance with federal and European guidelines and have been approved by university and state authorities. NOD-SCID mice (male, 12 weeks, weight between 20 and 25 g, 4 mice/group) received i.v. injections of 0.7 nmol of the sctb ds[19x16x19] (60µg) or the bsscFv ds[19x16] (40µg) in a total volume of 200 µl PBS or PBS alone. At various time points, blood samples (50-100 µl) were taken. Serum was prepared using BD Microtainer SST™ tubes (Becton Dickinson) and was stored at -20°C. Residual immunoreactive levels of the antibody fragments retained in serum were detected with Alexa Fluor 555 conjugated anti penta-His antibody (Qiagen), by flow cytometry using CD19-positive SEM and CD16-transfected CHO cells. The value obtained at the first time point (1 min) was set to 100%. A one-phase exponential decay curve fit was used to calculate the plasma half-life.

### Graphical and statistical analysis

Graphical and statistical analyses were performed using Graph Pad Prism Software (Graph Pad Software Inc., San Diego, CA, USA). Group data are reported as means ± standard error of the mean (SEM). Differences between groups were analysed using unpaired (or, where appropriate) paired Student's t-test. P-values < 0.05 were considered significant.

### Example 6: Construction, expression and purification of the tandem scFv triple-body (triabody) sctb ds[19x16x19]

The sctb ds[19x16x19] was constructed from previously published disulphide-stabilized scFv components with specificity for CD19 and CD16 (Bruenke et al, 2005, Fig. 2A). The two distal scFvs were specific for the lymphoid tumour antigen CD19, and the central scFv was specific for the trigger molecule CD16 on NK cells and macrophages. Both the sctb ds[19x16x19] and, for comparison, the bsscFv ds[19x16], were produced in stably transfected human 293T cells, cultured in a mini-PERM bioreactor, and purified from culture medium by affinity chromatography. Yields of each purified protein ranged from 3-10 mg per litre of culture medium in different production runs. The sctb ds[19x16x19] had an electrophoretic mobility in SDS-PAGE experiments corresponding to a Mᵣ of approximately 90 kDa, in close agreement with the value of 88.6 kDa predicted from its sequence (Fig. 2B). No immunoreactive degradation products were observed (Fig. 3C) and no covalent protein aggregates were detected in non-reducing SDS-PAGE experiments (data not shown).

This purified sctb ds[19x16x19] was used for the following experiments:

### Example 7: Specific and simultaneous antigen binding of the sctb ds[19x16x19]

The sctb ds[19x16x19] reacted with both CD19 and CD16 on intact cells, as demonstrated by its ability to specifically bind cells separately expressing each of these antigens (Fig. 3A). To exclude the possibility that the protein preparation consisted of two subpopulations of molecules each capable of binding only one of these antigens, the ability to simultaneously bind both antigens was analysed (Fig. 3B). To this effect a recombinant fusion protein between the extracellular domain of CD16 and GFP, termed CD16ex-GFP and, for comparison, a similar fusion protein consisting of the extracellular domain of CD64 and GFP, termed CD64ex-GFP, were used (Bruenke et al., 2005). CD19-positive leukaemia-derived SEM cells were incubated first with the sctb ds[19x16x19] and then reacted with CD16ex-GFP. As a result, a strong cell-bound fluorescence signal was observed indicating that the recombinant protein was capable of simultaneous binding to both cell-associated CD19 and fluid-phase CD16ex. No fluorescence signal was obtained when the CD7-negative SEM cells were incubated with the similarly produced control sctb [7xds16x7] or the control protein CD64ex-GFP (data not shown). Specificity of binding was established in competition-binding experiments. The intensity of the fluorescence signal was reduced to baseline levels when excess amounts of the parental CD19 antibody mAb 4G7 or the parental CD16 antibody mAb 3G8 were added, whereas the signal was not decreased after addition of a non-relevant IgG1 isotype control (Fig. 3B).

### Example 8: Comparison of the of the binding capacities of the sctb ds[19x16x19] and the bsscFv ds[19x16]

To assess, whether incorporation of a second scFv-component for the target-cell antigen in the sctb resulted in a gain of avidity for CD19, three independent experiments were performed. First, the equilibrium constants (K_{D}) of the sctb ds[19x16x19] and the bsscFv ds[19x16] were determined. Equilibrium binding curves for both molecules were recorded by calibrated flow cytometry, and K_{D} values were derived (Fig. 4A). The sctb ds[19x16x19] had an overall K_{D} of 13.0 ± 1.2 nM for CD19, while the affinity of the bsscFv ds[19x16] was approximately 3-fold lower: K_{D} = 42.4 ± 5.7 nM (P = 0.0048). Thus, the avidity of the entire sctb ds[19x16x19] for CD19 was approximately 3-fold higher than the affinity of the monovalent CD19-specific scFv contained in the bsscFv ds[19x16], indicating that both CD19-specific scFv components were functional and contributed to the overall avidity of the sctb ds[19x16x19] for CD19 on intact cells. In contrast, the affinity of the CD16 specific scFv remained unchanged, regardless of whether this component was carried in the bsscFv ds[19x16], where only its N-terminus was engaged, or in the sctb ds[19x16x19], where it was engaged at both ends by CD19 scFvs. The K_{D} values for CD16 of the sctb ds[19x16x19] and the bsscFv ds[19x16] were 58.6 ± 4.0 nM and 57.6 ± 8.9 nM, respectively (P = 0.772). Thus, the 3-fold increase in binding to CD19 was a genuine avidity effect, and both distally located CD19-specific scFvs in this format must have contributed to the overall avidity of the sctb ds[19x16x19] for the target cell surface antigen.

A second, independent experiment provided additional support for the claim that the sctb ds[19x16x19] had a greater avidity for CD19 than the bsscFv ds[19x16]. In a competition binding experiment, CD19-positive SEM cells were incubated first with the parental CD19 antibody mAb 4G7, then increasing amounts of either the sctb ds[19x16x19] or the bsscFv ds[19x16] were added as competitors, and residual binding of the mAb 4G7 to the cells was determined (Fig. 4B). Both the sctb ds[19x16x19] and the bsscFv ds[19x16] competitively inhibited binding of mAb 4G7 in a concentration-dependent manner, and binding was inhibited to > 95% by the addition of a 450-fold molar excess of the sctb ds[19x16x19]. To compare both constructs, IC₅₀ values were determined, i.e. the concentration, at which half-maximum inhibition was reached. The IC₅₀ values for the sctb ds[19x16x19] and the bsscFv ds[19x16] were 81.3 ± 1.2 nM and 287.5 ± 1.3 nM (P = 0.013), respectively. Hence, approximately 3.5-times greater concentrations of the bsscFv ds[19x16] than of the sctb ds[19x16x19] were needed to achieve half-maximum inhibition. This value is very close to the 3-fold difference in avidity, and therefore both independent measurements mutually confirm each other and support the claim of an approximately 3-fold increase in overall avidity for the sctb ds[19x16x19].

In a third, independent experiment, the cell surface-retention of both the sctb ds[19x16x19] and the bsscFv ds[19x16] on intact CD19-positive SEM cells at 37°C was measured. In this experiment, SEM cells were separately decorated with either the sctb ds[19x16x19] or the bsscFv ds[19x16], in the presence of sodium azide to prevent internalisation. Excess unbound molecules were removed by centrifugation and then the cells were incubated at 37 °C. At different time points, cells were again sedimented to remove released molecules and the remaining cell-bound molecules were determined by flow cytometry. Under these conditions, the bsscFv ds[19x16] was released from the surface with a retention t_{1/2} of 7.7 ± 0.5 min and the sctb ds[19x16x19] with a t_{1/2} of 51.5 ± 2.9 min (P = 0.006; Fig. 4C). No statistically significant differences were observed in cell surface retention of either construct by CD16 on CD16-positive cells (data not shown). The t_{1/2} values for retention by CD16 were 14.5 ± 1.9 min and 15.5 ± 1.2 min for the bsscFv and the sctb ds[19x16x19], respectively (P = 0.848). Taken together, these three independent experiments provide strong support for the conclusion that both CD19-specific scFv components contribute to the overall avidity of the sctb ds[19x16x19].

### Example 9: ADCC of leukaemia-derived cell lines mediated by the sctb ds[19x16x19]

The ability of the sctb ds[19x16x19] to mediate ADCC of human leukaemic cells in combination with effector cells from healthy human donors was measured first for the pro-B ALL derived cell line SEM. ADCC reactions were performed with freshly prepared unstimulated MNCs from unrelated healthy donors. As a result, the sctb ds[19x16x19] mediated effective tumor cell lysis, whereas a control sctb with specificities for CD7 and CD16 was ineffective (Fig. 5A; SEM cells are CD7-negative). Also, the parental antibodies mAb 4G7 (CD19) and mAb 3G8 (CD16) were unable to induce ADCC. To test for the antigen-specificity of lysis, competition experiments were performed. Addition of a 125-fold excess of the parental mAbs 4G7 or 3G8 significantly reduced the extent of target-cell lysis (P = 0.011 and 0.004, respectively), while addition of a non-relevant IgG1 isotype antibody had no effect (P = 0.45; Fig. 5A). Thus, tumour cell lysis was antigen-specific, and the sctb ds[19x16x19] required specific interaction with both the target antigen on the tumor cells and the trigger molecule on the effector cells to mediate its lytic effect. Moreover, the sctb ds[19x16x19] induced target cell lysis by ADCC over a broad range of effector-to-target cell (E:T) ratios, ranging from 80:1 to 2.5:1 (Fig. 5B). The extent of lysis increased with the E:T ratio in a saturable manner, demonstrating a clear dependence on the effector cells for tumor cell lysis. The effect was antigen-specific, because the similarly constructed control sctb with specificity for CD7 produced no significant lysis, not even at high E:T ratios.

### Example 10: Effectiveness of the tandem scFv triple-body (triabody) sctb ds [19x16x19] as compared to corresponding bispecific antibody bsscFv ds[19x16].

To investigate whether the increased avidity of the sctb ds[19x16x19] over the bsscFv ds[19x16] for CD19 would be associated with enhanced cytotoxic potential, the ability of both molecules to mediate ADCC was measured in parallel for three different CD19-positive tumour-derived cell lines. The cell lines SEM, BV-173 and ARH-77 were chosen, because they represented different B-lymphoid malignancies and different stages of B-lymphoid maturation. SEM cells represented a high-risk pro B-ALL. BV-173 represented a B-cell precursor leukaemia with mixed lineage phenotype (CD19 and CD33 double-positive), which often have poor prognosis. ARH-77 represented an EBV-positive B-lymphoblastoid cell-line. All three lines were efficiently eliminated by the sctb ds[19x16x19] and the bsscFv ds[19x16] in a concentration-dependent manner (Fig. 6). In all three cases the sctb ds[19x16x19] was more effective than the bsscFv ds[19x16], whereas the CD7-specific control sctb and bsscFv remained uneffective, even at high concentrations (Fig. 6, A-C). EC₅₀ values are shown in Table I; the EC₅₀ values for SEM cells for the sctb ds[19x16x19] and the bsscFv ds[19x16] were 4.1 pM and 113.6 pM, respectively. The EC₅₀ values obtained for the sctb were in the low picomolar range, which indicates a very high potency. Importantly, for the same target cell line the sctb ds[19x16x19] achieved an equal level of ADCC at 27.7-fold lower concentrations than the bsscFv ds[19x16]. Similarly, for the cell-lines BV-173 and ARH-77, the sctb ds[19x16x19] produced half-maximum lysis at 26.3- and 44.0-fold lower concentrations than the bsscFv ds[19x16], indicating that the gain in avidity of the sctb over the bsscFv translated into a significant gain in ADCC-potential.

**Table I: EC₅₀ values for ADCC by sctb ds[19x16x19] and bsscFv ds[19x16] with MNCs^{a}**

| target cells | sctb [pM] (95% Cl) | bsscFv [pM] (95% Cl) | fold difference | P-value for difference of log EC₅₀ |
|---|---|---|---|---|
| cell lines | | | | |
| SEM | 4.1 (2.8-6.0) | 113.6 (24.5-526.3) | 27.7 | 0.004 |
| BV-173 | 28.6 (18.0-45.3) | 753.3 (445.4-1,274) | 26.3 | 0.001 |
| ARH-77 | 29.0 (17.7-47.6) | 1,277 (587.2-2,777) | 44.0 | 0.006 |
| | | | | |

| primary malignant cells | | | | |
|---|---|---|---|---|
| patient 1 | 20.4 (9.2-44.9) | 416.4 (277.1-625.8) | 20.4 | 0.028 |
| patient 2 | 104.5 (31.2-350.2) | 2,117 (964.9-4,646) | 20.3 | 0.009 |
| patient 3 | 37.2 (18.2-77.6) | 425.4 (202.4-894.4) | 11.4 | 0.014 |
| patient 4 | 13.7 (6.4-26.6) | 294.3 (154.3-561.3) | 21.5 | < 0.001 |

| | | | | |
|---|---|---|---|---|
| ^{a}EC₅₀ values were deduced from the dose-response curves shown in Fig. 6A-C and 7 B (Cl: confidence interval) | | | | |

Finally, the ability of both proteins to mediate the elimination of fresh malignant cells from patients was also measured in parallel ADCC reactions (Fig. 7). To this effect, MNCs were isolated from either the peripheral blood (PB) or the bone-marrow (BM) of eleven patients. Of these patients nine were diagnosed with B-CLL, one with mantle cell lymphoma (MCL), and one with B-ALL. MNCs from unrelated healthy donors were used as effector cells, one donor for each patient sample. No deliberate effort was made to match the histocompatibility type and killer cell Ig-like receptor (KIR) patterns of tumor cells and donors. MNCs were used at an E:T ratio of 40:1 and both the sctb ds[19x16x19] and the bsscFv ds[19x16] at a concentration of 1 nM. For all eleven patients the sctb ds[19x16x19] mediated a clearly enhanced extent of specific tumor cell lysis relative to control reactions containing either no added antibody-derived agent or a non-relevant control sctb (Fig. 7A). For all eleven samples, the sctb ds[19x16x19] consistently produced greater specific target-cell lysis than the bssFv ds[19x16]. For four B-CLL patients (patients 1 - 4) dose-response-curves were recorded in at least three separate experiments using MNCs from at least three different healthy donors for each patient sample at an E:T ratio of 40:1. For all four patients the sctb ds[19x16x19] uniformly mediated significantly greater ADCC than the bsscFv ds[19x16] at equimolar concentrations (Fig. 7B). The EC₅₀ values for all four patients for both the sctb ds[19x16x19] and the bsscFv ds[19x16] are summarized in Table I. In all cases the sctb ds[19x16x19] produced half-maximum lysis at approximately 11- to 21-fold lower concentrations than the bsscFv ds[19x16], similar to the effects observed with leukeamia-derived target lines (Fig. 6). The EC₅₀ values for the sctb ds[19x16x19] were again in the low picomolar range. In all cases, the differences of lytic ability between the sctb ds[19x16x19] and the bsscFv ds[19x16] were statistically significant, with P-values < 0.05. These results clearly demonstrate that the format of a sctb has a superior potential to induce tumor cell lysis than the respective bsscFv.

### Example 11: Prolonged in vivo plasma retention of the sctb ds[19x16x19] in mice

Another important parameter influencing the therapeutic value of antibody-derived proteins is their *in vivo* plasma retention time or plasma half-life. To measure this parameter, equimolar doses of the sctb ds[19x16x19] and the bsscFv ds[19x16] were injected separately i.v. into NOD-SCID mice. Plasma samples were obtained at various time points after injection, and the residual binding ability for CD19 on CD19-positive SEM cells (Fig. 8A) and for CD16 on CD16-positive CHO-transfectants (Fig. 8B) was measured by flow cytometry. The sctb ds[19x16x19] and the bsscFv ds[19x16] had plasma retention times (t_{1/2}) of 4 h and 2 h, respectively, indicating that the incorporation of a second CD19-specific scFv into the sctb caused a doubling of the *in vivo* plasma retention time.

### Example 12: Measurement of in vitro serum stability at 37 °C

An important factor affecting the therapeutic efficacy of antibody-derived proteins is their stability in human serum. To measure this property, the purified sctb ds[19x16x19] was incubated in human serum at 37°C *in vitro,* and residual binding to CD19 (Fig. 9A) and CD16 (Fig. 9B), respectively, on intact cells was measured by FACS-analysis. The values for t_{1/2} were determined. The sctb ds[19x16x19] was highly stable under these conditions with t_{1/2} of 94.0 ± 3.1 h and 153.1 ± 4.6 h for binding to CD19 and CD16, respectively. Therefore the sctb ds[19x16x19] had an higher stability than the non-stabilized bsscFv [19x16], for which t_{1/2} of 18 h and 40 h for binding to CD19 and CD16, respectively, were reported in Bruenke et al., 2005.

### Example 13: Construction and expression of sctb ds[33xds16x33] and bsscFv [33xds16].

The sctb [33xds16x33] (Fig. 10) and the bsscFv [33xds16] (Fig. 11) were constructed from previously published scFv components with specificity for CD33 (Schwemmlein et al, 2005). Both the sctb ds[33xds16x33] and, for comparison, the bsscFv [33xds16], were produced in stably transfected human 293T cells being cultured in a mini-PERM bioreactor. The recombinant proteins were purified from culture medium by affinity chromatography. Yields of each purified protein ranged from 2-6 mg per litre of culture medium. The sctb [33xds16x33] and the bsscFv [33xds16] had an electrophoretic mobility in SDS-PAGE experiments corresponding to a Mᵣ of about 90 kDa and 60 kDa respectively, in close agreement with the value of 86,5 kDa for the sctb [33xds16x33] (Fig. 10) and 59,8 kDa for the bsscFv [33xds16] (Fig. 11) calculated from their sequences.

### Example 14: Specific and simultaneous antigen binding of the sctb [33xds16x33] and the bsscFv [33xds16].

Both the sctb [33xds16x33] (Fig. 12A) and the bsscFv [33xds16] (Fig. 13A) reacted with both the myeloid tumour antigen CD33 and the trigger molecule CD16 on intact cells, as it was capable of specifically binding to cells separately expressing each of these antigens. To ascertain that the protein was capable of binding to both antigens simultaneously, CD16-positive cells were incubated first with the sctb [33xds16x33] (Fig. 12B) or the bsscFv [33xds16] (Fig. 13B) and then reacted with a fusion protein consisting of the extracellular domain of CD33 and DsRed CD33ex-DsRed. As a result, a strong red, cell-bound fluorescence signal was observed indicating that the recombinant protein was able to react simultaneously with both cell-associated CD16 and fluid-phase CD33ex.

### Example 15: Determination of equilibrium constants

To assess, whether incorporation of a second scFv-component for the target-cell antigen in the sctb resulted in a gain of avidity for CD33, the equilibrium constants (K_{D}) of the sctb [33xds16x33] and the bsscFv [33xds16] were determined. The K_{D} values for both proteins were derived from equilibrium binding curves that were recorded for both antigens by calibrated flow cytometry (Fig. 14). The K_{D} -values are summarized in table II. The sctb [33xds16x33] and the bsscFv [33xds16] reacted with CD33 with K_{D} -values of 28.9 ± 1,9 nM and 7.9 ± 1.1 nM, respectively. Thus, the avidity of the sctb [33xds16x33] for CD33 was approximately 3-4-fold higher than the affinity of the CD33-specific scFv contained in the bsscFv [33xds16]. This suggests that both CD33-specific scFv moieties were functional and contributed to binding to CD33 on intact cells. In contrast, the affinities of the CD16 specific scFv were in the same range. The K_{D} values for CD16 of the sctb [33xds16x33] and the bsscFv [33xds16] were 45.1 ± 4.3 nM and 34.2 ± 2.3 nM, respectively. Thus, both distally located CD33-specific scFvs in this format contributed to the overall avidity of the sctb [33xds16x33] for the cell surface antigen on the tumour cells.

**Table II: Equilibrium constants for the bsscFv [33xds16] and the sctb [33xds16x33]**

| antigen (Cell line) | K_{D} of bsscFv [33xds16] [nM] (SEM) | K_{D} of sctb [33xds16x33] [nM] (SEM) |
|---|---|---|
| CD33 (HL-60) | 28,9 (±1,9) | 7,9 (±1,1) |
| CD16 (CHO 16-10) | 35,3 (± 2,2) | 45,1 (± 4,3) |

### Example 16: Effectiveness of the tandem scFv triple-body (triabody) sctb ds [33xds16x33] as compared to corresponding bispecific antibody bsscFv [33xds16].

To analyze whether the enhanced avidity of the sctb [33xds16x33] over the bsscFv [33xds16] for CD33 would result in an increased cytolytic potential, the efficacy of both antibody fragments to induce ADCC was measured in parallel. The CD33-positive promyelocytic leukemia -derived tumour cell line HL-60 was used as target to compare the sctb and the bsscFv. MNCs from healthy donors were used as effector cells at a constant E:T cell ratio of 40:1. The sctb [33xds16x33] and the bsscFv [33xds16] triggered ADCC in a dose-dependent manner, whereas the CD7-specific control sctb and bsscFv remained uneffective, even at high concentrations. However, the sctb [33xds16x33] was more effective than the bsscFv [33xds16] (Fig. 15). The EC₅₀ values for HL-60 cells for the sctb [33xds16x33] and the bsscFv [33xds16] were 21 pM and 174 pM, respectively (p=0,018). Importantly, for the same target cell line the sctb [33xds16x33] produces half maximum killing at 8-fold lower concentrations than the bsscFv [33xds16], suggesting that the gain in avidity of the sctb over the bsscFv translated into a significant gain in the cytotoxic potential.

### References:

Ausubel, Current Protocols in Molecular Biology, Green Publishing Associates and Wiley Interscience, N.Y. (2001).
Adams GP, Schier R, McCall AM, Crawford RS, Wolf EJ, Weiner LM, Marks JD. Prolonged in vivo tumour retention of a human diabody targeting the extracellular domain of human HER2/neu. Br J Cancer. 1998; 77: 1405-1412.
Barbin K, Stieglmaier J, Saul D, Stieglmaier K, Stockmeyer B, Pfeiffer M, Lang P, Fey GH. Influence of variable N-glycosylation on the cytolytic potential of chimeric CD19 antibodies. J Immunother (1997). 2006;29:122-133.
Batra JK, Kasprzyk PG, Bird RE, Pastan I, King CR. Recombinant anti-erbB2 immunotoxins containing Pseudomonas exotoxin. Proc Natl Acad Sci USA. 1992;89:5867-5871.
Bebbington CR, Renner G, Thomson S, King D, Abrams D, Yarranton GT. High-level expression of a recombinant antibody from myeloma cells using a glutamine synthetase gene as an amplifiable selectable marker. Bio/Technology 1992; 10:169-175.
Benedict CA, MacKrell AJ, Anderson WF. Determination of the binding affinity of an anti-CD34 single-chain antibody using a novel, flow cytometry based assay. J Immunol Methods. 1997; 201(2):223-31.
Bonnet D and Dick JE. Human acute myeloid leukemia is organized as a hierarchy that originates from a primitive hematopoietic cell. Nat Med. 1997; 3(7): 730-7.
Braasch DA and Corey DR. Locked nucleic acid (LNA): fine-tuning the recognition of DNA and RNA. Chem Biol. 2001; 8(1):1-7.
Brinkmann U, Lee BK and Pastan I. Recombinant Immunotoxins Containing the VH or VL Domain of Monoclonal Antibody B3 fused to Pseudomonas Exotoxin. J. Immunology. 1993; 150:2774-2782.
Bruenke J, Barbin K, Kunert S, Lang P, Pfeiffer M, Stieglmaier K, Niethammer D, Stockmeyer B, Peipp M, Repp R, Valerius T and Fey GH. Effective lysis of lymphoma cells with a stabilised bispecific single-chain Fv antibody against CD19 and FcγRIII (CD16). British Journal of Haematology. 2005; 130:218-228.
Bruenke J, Fischer B, Barbin K, Schreiter K, Wachter Y, Mahr K, Titgemeyer F, Niederweis M, Peipp M, Zunino SJ, Repp R, Valerius T, Fey GH. A recombinant bispecific single-chain Fv antibody against HLA class II and FcgammaRIII (CD16) triggers effective lysis of lymphoma cells. Brit J Haematol. 2004; 125(2): 167-79.
Bryder D, Rossi DJ, Weissman IL. Hematopoietic Stem Cells. Am J Pathol.2006; 169 (2): 338-46.
Carnahan J, Wang P, Kendall R, Chen C, Hu S, Boone T, Juan T, Talvenheimo J, Montestruque S, Sun J, Elliott G, Thomas J, Ferbas J, Kern B, Briddell R, Leonard JP, Cesano A. Epratuzumab, a humanized monoclonal antibody targeting CD22: characterization of in vitro properties. Clin Cancer Res. 2003;9:3982S-3990S.
Cartron G, Dacheux L, Salles G, Solal-Celigny P, Bardos P, Colombat P, Watier H. Therapeutic activity of humanized anti-CD20 monoclonal antibody and polymorphism in IgG Fc receptor FcgammaRIIIa gene. Blood. 2002;99:754-758.
Casasnovas RO, Slimane FK, Garant R, Faure GC, Campos L, Deneys V, Bernier M, Falkenrodt A, Lecalvez G, Maynadie M and Dene MC. Immunological classification of acute myeloplastic leukaemias: relevance to patient outcome. Leukemia. 2003; 17: 515-527.
Castor A, Nilsson L, Astrand-Grundstrom I, Buitenhuis M, Ramirez C, Anderson K, Strombeck B, Garwicz S, Bekassy AN, Schmiegelow K, Lausen B, Hokland P, Lehmann S, Juliusson G, Johansson B, Jacobsen SE. Distinct patterns of hematopoietic stem cell involvement in acute lymphoblastic leukemia. Nat Med. 2005;11:630-637.
Cheung MC, Haynes AE, Stevens A, Meyer RM, Imrie K. Yttrium 90 ibritumomab tiuxetan in lymphoma. Leuk Lymphoma. 2006;47:967-977.
Clynes RA, Towers TL, Presta LG, Ravetch JV. Inhibitory Fc receptors modulate in vivo cytoxicity against tumor targets. Nat Med. 2000;6:443-446.
Davies AJ. Radioimmunotherapy for B-cell lymphoma: Y90 ibritumomab tiuxetan and I(131) tositumomab. Oncogene. 2007;26:3614-3628.
de Palazzo IG, Kitson J, Gercel-Taylor C, Adams S, Weiner LM. Bispecific monoclonal antibody regulation of Fc gamma RIII-directed tumor cytotoxicity by large granular lymphocytes. Cell Immunol. 1992;142:338-347.
Elsasser D, Valerius T, Repp R, Weiner GJ, Deo Y, Kalden JR, van de Winkel JG, Stevenson GT, Glennie MJ, Gramatzki M. HLA class II as potential target antigen on malignant B cells for therapy with bispecific antibodies in combination with granulocyte colony-stimulating factor. Blood. 1996; 87: 3803-12.
Ely P, Wallace PK, Givan AL, Graziano RF, Guyre PM, Fanger MW. Bispecific-armed, interferon gamma-primed macrophage-mediated phagocytosis of malignant non-Hodgkin's lymphoma. Blood. 1996;87:3813-3821.
Eyrich M, Lang P, Lal S, Bader P, Handgretinger R, Klingebiel T, Niethammer D, Schlegel PG. A prospective analysis of the pattern of immune reconstitution in a paediatric cohort following transplantation of positively selected human leucocyte antigen-disparate haematopoietic stem cells from parental donors. Br J Haematol. 2001;114:422-432.
Ghahroudi MA, Desmyter A, Wyns L, Hamers R and Muyldermans S. Selection and identification of single domain antibody fragments from camel heavy-chain antibodies. FEBS Letters 1997; 414:521-526.
Glockshuber R, Malia M, Pfitzinger I, Pluckthun A. A comparison of strategies to stabilize immunoglobulin Fv-fragments. Biochemistry. 1990;29:1362-1367.
Greil J, Gramatzki M, Burger R, Marschalek R, Peltner M, Trautmann U, Hansen-Hagge TE, Bartram CR. Fey GH, Stehr K, et al. The acute lymphoblastic leukaemia cell line SEM with t(4;11) chromosomal rearrangement is biphenotypic and responsive to interleukin-7. Br J Haematol. 1994; 86(2):275-83.
Grossbard ML, Press OW, Appelbaum FR, Bernstein ID, Nadler LM. Monoclonal antibody-based therapies of leukemia and lymphoma. Blood. 1992;80:863-878.
Haagen IA, van deGriend R, Clark M, Geerars A, Bast B, deGast B. Killing of human leukaemia/lymphoma B cells by activated cytotoxic T lymphocytes in the presence of a bispecific monoclonal antibody (α-CD3/α-CD19). Clin. Exp. Immunol. 1992; 90: 368-375.
Hale G, Dyer MJ, Clark MR, Phillips JM, Marcus R, Riechmann L, Winter G, Waldmann H. Remission induction in non-Hodgkin lymphoma with reshaped human monoclonal antibody CAMPATH-1H. Lancet. 1988;2:1394-1399.
Hamann PR, Hinman LM, Beyer CF, Lindh D, Upeslacis J, Flowers DA, Bernstein I. An anti-CD33 antibody-calicheamicin conjugate for treatment of acute myeloid leukemia. Choice of linker. Bioconjug Chem. 2002;13:40-46.
Harlow and Lane "Antibodies, A Laboratory Manual", Cold Spring Harbor Laboratory Press, 1988.
Harlow and Lane "Using Antibodies: A Laboratory Manual" Cold Spring Harbor Laboratory Press, 1999.
Hartmann F, Renner C, Jung W, Deisting C, Juwana M, Eichentopf B, Kloft M, Pfreundschuh M. Treatment of refractory Hodgkin's disease with an anti-CD16/CD30 bispecific antibody. Blood. 1997;89:2042-2047.
Hekman A, Honselaar A, Vuist WM, Sein JJ, Rodenhuis S, ten Bokkel Huinink WW, Somers R, Rumke P, Melief CJ. Initial experience with treatment of human B cell lymphoma with anti-CD19 monoclonal antibody. Cancer Immunol Immunother. 1991;32:364-372.
Hombach A, Jung W, Pohl C, Renner C, Sahin U, Schmits R, Wolf J, Kapp U, Diehl V, Pfreundschuh M. A CD16/CD30 bispecific monoclonal antibody induces lysis of Hodgkin's cells by unstimulated natural killer cells in vitro and in vivo. Int J Cancer. 1993;55:830-836.
Hosen N, Park CY, Tatsumi N, Oji Y, Sugiyama H, Gramatzki M, Krensky AM, Weissman IL. CD96 is a leukemic stem cell-specific marker in human acute myeloid leukemia. Proc Natl Acad Sci USA. 2007, 104(26):11008-13.
Hrusak O and Porwit-MacDonald A. Antigen expression patterns reflecting genotype of acute leukaemias. Leukemia. 2002; 16(7):1233-1258.
Jin L, Hope KJ, Zhai Q, Smadja-Joffe F, Dick JE. Targeting of CD44 eradicates human myeloid leukemic stem cells. Nat. Med. 2006; 12(10): 1167-74.
Kabat EA, Wu TT. Identical V region amino acid sequences and segments of sequences in antibodies of different specificities. Relative contributions of VH and VL genes, minigenes, and complementarity-determining regions to binding of antibody-combining sites. J Immunol. 1991;147:1709-1719.
Kaminski MS, Zasadny KR, Francis IR, Milik AW, Ross CW, Moon SD, Crawford SM, Burgess JM, Petry NA, Butchko GM, et al. Radioimmunotherapy of B-cell lymphoma with [131I]anti-B1 (anti-CD20) antibody. N Engl J Med. 1993;329:459-465.
Kipriyanov SM, Moldenhauer G, Strauss G and Little M. Bispecific CD3XCD19 diabody for T cell-mediated lysis of malignant human B cells. Int. J. Cancer. 1998; 77: 763-772.
Kipriyanov SM, Cochlovius B, Schafer HJ, Moldenhauer G, Bahre A, Le Gall F, Knackmuss S, Little M. Synergistic antitumor effect of bispecific CD19 x CD3 and CD19 x CD16 diabodies in a preclinical model of non-Hodgkin's lymphoma. J Immunol. 2002;169:137-144.
Krebber A, Bornhauser S, Burmester J, Honegger A, Willuda J, Bosshard HR, Pluckthun A. Reliable cloning of functional antibody variable domains from hybridomas and spleen cell repertoires employing a reengineered phage display system. J Immunol Methods. 1997; 201(1):35-55.
Laemmli UK. Cleavage of structural proteins during the assembly of the head of bacteriophage T4. Nature. 1970; 227(5259): 680-5.
Lang P, Handgretinger R, Niethammer D, Schlegel PG, Schumm M, Greil J, Bader P, Engel C, Scheel-Walter H, Eyrich M, Klingebiel T. Transplantation of highly purified CD34+ progenitor cells from unrelated donors in pediatric leukemia. Blood. 2003;101:1630-1636.
Lapidot T, Sirard C, Vormoor J, Murdoch B, Hoang T, Caceres-Cortes J, Minden M, Paterson B, Caligiuri MA, Dick JE. Nature. 1994; 367 (6464): 645-8.
Lazar GA, Dang W, Karki S, Vafa O, Peng JS, Hyun L, Chan C, Chung HS, Eivazi A, Yoder SC, Vielmetter J, Carmichael DF, Hayes RJ, Dahiyat BI. Engineered antibody Fc variants with enhanced effector function. Proc Natl Acad Sci USA. 2006; 103: 4005-4010.
Legras S, Gunthert U, Stauder R, Curt F, Oliferenko S, Kluin-Nelemans HC, Marie JP, Proctor S, Jasmin C, Smadja-Joffe F. A strong expression of CD44-6v correlates with shorter survival of patients with acute myeloid leukemia. Blood. 1998;91:3401-3413.
Leonard JP, Coleman M, Ketas JC, Chadburn A, Ely S, Furman RR, Wegener WA, Hansen HJ, Ziccardi H, Eschenberg M, Gayko U, Cesano A, Goldenberg DM. Phase I/II trial of epratuzumab (humanized anti-CD22 antibody) in indolent non-Hodgkin's lymphoma. J Clin Oncol. 2003;21:3051-3059.
Loffler A, Kufer P, Lutterbuse R, Zettl F, Daniel PT, Schwenkenbecher JM, Riethmuller G, Dorken B, Bargou RC. A recombinant bispecific single-chain antibody, CD19 x CD3, induces rapid and high lymphoma-directed cytotoxicity by unstimulated T lymphocytes. Blood. 2000;95:2098-2103.
McCall, A.M., Shahied, L., Amoroso, A.R., Horak, E.M., Simmons, H.H., Nielson, U., Adams, G.P., Schier, R., Marks, J.D. & Weiner, L.M. (2001) Increasing the affinity for tumor antigen enhances bispecific antibody cytotoxicity. J Immunol, 166, 6112-6117.
Messinger Y, Yanishevski Y, Ek O, Zeren T, Waurzyniak B, Gunther R, Chelstrom L, Chandan-Langlie M, Schneider E, Myers DE, Evans W, Uckun FM. In vivo toxicity and pharmacokinetic features of B43 (anti-CD19)-genistein immunoconjugate in nonhuman primates. Clin Cancer Res. 1998;4:165-170.
Murphy G, Cockett MI, Ward RV and Docherty AJ. Matrix metalloproteinase degradation of elastin, type IV collagen and proteoglycan. A quantitative comparison of the activities. Biochem J. 1991; 277:277-279.
Onrust SV, Lamb HM, Balfour JA. Rituximab. Drugs. 1999;58:79-88; discussion 89-90.
Owens GC., Chappell SA., Mauro VP. and Edelman GM. Identification of two short internal ribosome entry sites selected from libraries of random oligonucleotides. Proc. Natl. Acad. Sci. USA 2001; 98(4): 1471-1476.
Pantoliano MW, Bird RE, Johnson S, Asel ED, Dodd SW, Wood JF, Hardman KD. Conformational stability, folding, and ligand-binding affinity of single-chain Fv immunoglobulin fragments expressed in Escherichia coli. Biochemistry. 1991;30:10117-10125.
Passegue E, Jamieson, CHM, Ailles LE, Weissman IL. Normal and leukemic hematopoiesis: Are leukemias a stem cell disorder or a reacquisition of stem cell characteristics? Proc Natl Acad Sci USA. 2003; 100 (suppl.1) 11842-49.
Peipp M, Valerius T. Bispecific antibodies targeting cancer cells. Biochem Soc Trans. 2002;30:507-511.
Peipp M, Kupers H, Saul D, et al. A recombinant CD7-specific single-chain immunotoxin is a potent inducer of apoptosis in acute leukemic T cells. Cancer Res. 2002; 62: 2848-2855.
Pietersz GA, Wenjun L, Sutton VR, Burgess J, McKenzie IF, Zola H, Trapani JA. In vitro and in vivo antitumor activity of a chimeric anti-CD19 antibody. Cancer Immunol Immunother. 1995;41:53-60.
Ravetch JV, Kinet JP. Fc receptors. Annu Rev Immunol. 1991;9:457-492.
Reff ME, Carner K, Chambers KS, Chinn PC, Leonard JE, Raab R, Newman RA, Hanna N, Anderson DR. Depletion of B cells in vivo by a chimeric mouse human monoclonal antibody to CD20. Blood. 1994;83:435-445.
Reiter Y, Brinkmann U, Kreitman RJ, Jung SH, Lee B and Pastan I. Stabilization of the Fv Fragments in Recombinant Immunotoxins by Disulfide Bonds Engineered into Conserved Framework Regions. Biochemistry. 1994; 33:5451-5459.
Riechmann L, Clark M, Waldmann H, Winter G. Reshaping human antibodies for therapy. Nature. 1988;332:323-327.
Sambrook, Molecular Cloning A Laboratory Manual, Cold Spring Harbor Laboratory (2001) N.Y.
Schwemmlein M, Stieglmaier J, Kellner C, Peipp M, Saul D, Oduncu F, Emmerich B, Stockmeyer B, Lang P, Beck JD, Fey GH. A CD19-specific single-chain immunotoxin mediates potent apoptosis of B-lineage leukemic cells. Leukemia. 2007;21:1405-1412.
Schwenkert M, Birkholz K, Schwemmlein M, Kellner C, Kügler M, Peipp M, Nettelbeck D M, Schuler-Thurner B, Schaft N, Dörrie J, Ferrone S, Kämpgen E, Fey GH. A single chain immunotoxin, targeting the melanoma associated chondroitin sulfate proteoglycan, is a potent inducer of apoptosis in cultured human melanoma cells. Melanoma Res. 2008 ;18(2):73-84.
Segal DM, Weiner GJ, Weiner LM. Bispecific antibodies in cancer therapy. Curr Opin Immunol. 1999;11:558-562.
Sievers EL, Appelbaum FR, Spielberger RT, Forman SJ, Flowers D, Smith FO, Shannon-Dorcy K, Berger MS, Bernstein ID. Selective ablation of acute myeloid leukemia using antibody-targeted chemotherapy: a phase I study of an anti-CD33 calicheamicin immunoconjugate. Blood. 1999;93:3678-3684.
Spangrude GJ, Heimfeld S, Weissman IL. Purification and characterization of mouse hematopoietic stem cells. Science. 1988;241:58-62..
Stemmer WP, Morris SK, Wilson BS. Selection of an active single chain Fv antibody from a protein linker library prepared by enzymatic inverse PCR. Biotechniques. 1993;14:256-265.
Stork R, Zettlitz KA, Müller D, Rether M, Hanisch FG, Kontermann RE. N-Glycosylation as Novel Strategy to Improve Pharmacokinetic Properties of Bispecific Single-chain Diabodies. J Biol Chem. 2008;283(12):7804-12.
Tan BT, Park CY, Ailles E, Weissman IL. The cancer stem cell hypothesis: a work in progress. Lab Invest. 2006; 86: 1203-7.
Taussig DC, Pearce DJ, Simpson C, Rohatiner AZ, Lister TA, Kelly G, Luongo JL, Danet-Desnoyers GH, Bonnet D. Hematopoietic stem cells express multiple myeloid markers: implications for the origin and targeted therapy of acute myeloid leukemia. Blood. 2005;106(13):4086-92.
Vallera DA, Elson M, Brechbiel MW, Dusenbery KE, Burns LJ, Jaszcz WB, Ramsay NK, Panoskaltsis-Mortar A, Kuroki DW, Wagner JE, Vitetta ES, Kersey JH. Radiotherapy of CD19 expressing Daudi tumors in nude mice with Yttrium-90-labeled anti-CD19 antibody. Cancer Biother Radiopharm. 2004;19:11-23.
van de Winkel JG, Anderson CL. Biology of human immunoglobulin G Fc receptors. J Leukoc Biol. 1991;49: 511-524.
van Rhenen A, van Dongen GAM, Kelder A1, Rombouts EJ, Feller N, Moshaver B, Stigter-van Walsum M, Zweegman S, Ossenkoppele GJ, Gerrit, Schuurhuis GJ. The novel AML stem cell-associated antigen CLL-1 aids in discrimination between normal and leukemic stem cells. Blood, 2007; 110, 7: 2659-2666.
Vervoordeldonk SF, Merle PA, van Leeuwen EF, von dem Borne AE, Slaper-Cortenbach IC. Preclinical studies with radiolabeled monoclonal antibodies for treatment of patients with B-cell malignancies. Cancer. 1994;73:1006-1011.
Weiner GJ, De Gast GC. Bispecific monoclonal antibody therapy of B-cell malignancy. Leuk Lymphoma. 1995;16:199-207.
Weiner LM, Holmes M, Richeson A, Godwin A, Adams GP, Hsieh-Ma ST, Ring DB, Alpaugh RK. Binding and cytotoxicity characteristics of the bispecific murine monoclonal antibody 2B1. J Immunol. 1993; 151: 2877-2886.
Weng WK, Levy R. Two immunoglobulin G fragment C receptor polymorphisms independently predict response to rituximab in patients with follicular lymphoma. J Clin Oncol. 2003;21:3940-3947.

## Claims

1. A nucleic acid molecule encoding a polypeptide,
wherein the polypeptide consists of
(a) a first immunoglobulin domain consisting of a VL domain linked to a VH domain, wherein the immunoglobulin domain specifically binds to an antigen expressed on tumour cells;
(b) a second immunoglobulin domain consisting of a VL domain linked to a VH domain, wherein the immunoglobulin domain specifically binds to an antigen expressed on tumour cells; and
(c) a third immunoglobulin domain consisting of a VL domain linked to a VH domain, wherein the immunoglobulin domain specifically binds to an effector cell antigen, wherein the effector cell is selected from the group consisting of NK cells, T cells, neutrophilic granulocytes, monocytes and macrophages,
wherein at least one of the antigens expressed on tumour cells and bound by the immunoglobulin domain of (a) or (b) is an antigen expressed on tumour stem cells and/or on tumour precursor or progenitor cells; and
(d) optionally, at least one (poly)peptide unrelated to immunoglobulin domains;
and wherein, the nucleic acid molecule encodes at least one linker which separates at least one V_{H} from a V_{L} domain or at least one V_{L} from a V_{H} domain.

2. The nucleic acid molecule of claim 1, wherein the immunoglobulin domains of items (a) and (b) bind to the same antigen expressed on tumour cells.

3. The nucleic acid molecule of claim 1, wherein the immunoglobulin domains of items (a) and (b) bind to different antigens expressed on tumour cells.

4. The nucleic acid molecule of any one of claims 1 to 3, wherein the at least one antigen expressed on tumour stem cells or on tumour precursor or progenitor cells is CD19, CD33, CD13, CD44var, CD123, CD96 or CLL-1.

5. The nucleic acid molecule of any one of claims 1 to 4, wherein the antigens expressed on tumour cells and bound by the immunoglobulin domains of (a) and (b) are CD19 and CD33, CD19 and CD13, CD19 and CD123, CD19 and CD44var, CD19 and MCSP, CD33 and CD13, CD33 and CD123, CD33 and CD44var, CD33 and CD96, CD33 and CLL-1, CD13 and CD123, CD13 and CD44var, CD13 and CD96, CD13 and CLL-1, CD123 and CD44var, CD123 and CD96, CD123 and CLL-1, CD44var and CD96, CD44var and CLL-1, or CD96 and CLL-1.

6. The nucleic acid molecule of any one of claims 1 to 5, wherein the effector cell antigen bound by the immunoglobulin domain of (c) is CD16 (FcγRIIIa), CD64 (FcγRI), NKG2D, NKp30, NKp44, NKp46, CD28, CD2 or CD3.

7. The nucleic acid molecule of any one of claims 1 to 6, wherein at least one immunoglobulin domain comprises at least one cysteine residue capable of forming intramolecular disulfide bridges.

8. The nucleic acid molecule of any one of claims 1 to 7, wherein the linker is a peptide linker of at least 5 amino acids.

9. The nucleic acid of claim 8, wherein the linkers are of uniform length if more than one linker is present.

10. The nucleic acid of claim 8, wherein the linkers are of different length if more than one linker is present.

11. The nucleic acid of claim 9, wherein the amino acid sequences of the linkers are identical.

12. The nucleic acid of claim 9, wherein the amino acid sequences of the linkers are different.

13. The nucleic acid of any one of claim 1 to 12 encoding two immunoglobulin domains specifically binding to CD19 and one immunoglobulin domain specifically binding to CD16 (FcγRIIIa).

14. The nucleic acid of any one of claim 1 to 12 encoding two immunoglobulin domains specifically binding to CD33 and one immunoglobulin domain specifically binding to CD16 (FcγRIIIa).

15. The nucleic acid molecule of claim 1, wherein the (poly)peptide unrelated to immunoglobulin domains is a Strep-tag, a His-tag, a Myc-tag, a Flag-tag, a kappa secretion leader, human serum albumin (hsa) or fragments thereof, peptides capable of binding to hsa or other serum proteins; a peptide capable of binding to neonatal Fc receptor (FcRn), human muscle aldolase (hma) or a fragment thereof, CD8 hinge region, Interleukin-2, Interleukin-15, Interleukin-18, Granulocyte-Macrophage-Colony Stimulating Factor (GM-CSF), Granulocyte-Colony Stimulating Factor (G-CSF) or a peptide providing at least one N-glycosylation site.

16. The nucleic acid molecule of any one of claims 1 to 15, wherein the tumour cells are leukaemia cells.

17. The nucleic acid molecule of any one of claims 1 to 15, wherein the tumour stem cells are leukaemic stem cells.

18. The nucleic acid molecule of any one of claims 1 to 15, wherein the tumour precursor or progenitor cells are leukaemic precursor or progenitor cells.

19. The nucleic acid molecule of any one of claims 1 to 18, wherein the tumour is a leukaemia or lymphoma.

20. A vector comprising the nucleic acid molecule of any one of claims 1 to 19.

21. A non-human host transformed with the vector of claim 20.

22. The non-human host of claim 21, wherein the host is a cell.

23. A method for the production of a polypeptide comprising culture of the host cell of claim 22 under suitable conditions and isolation of the recombinant polypeptide produced.

24. A polypeptide encoded by the nucleic acid molecule of any one of claims 1 to 19 or produced by the method of claim 23.

25. A diagnostic composition comprising at least one of
(a) the nucleic acid molecule of any one of claims 1 to 19
(b) the vector of claim 20 or
(c) the polypeptide of claim 24.

26. A pharmaceutical composition comprising
(a) the nucleic acid molecule of any one of claims 1 to 19
(b) the vector of claim 20 or
(c) the polypeptide of claim 24.

27. The nucleic acid molecule of any one of claims 1 to 19, the vector of claim 20 or the polypeptide of claim 24 for use in the treatment of tumours.

28. The nucleic acid molecule or the vector or the polypeptide for use acording to claim 27, wherein the tumour is a leukaemia or lymphoma.

29. The nucleic acid molecule or the vector or the polypeptide for use according to claim 28, wherein the leukaemia is AML, CML, ALL, CLL or non-Hodgkin lymphoma.

## Patentansprüche

1. Nukleinsäuremolekül, das ein Polypeptid kodiert,
wobei das Polypeptid aus
(a) einer ersten Immunglobulin-Domäne bestehend aus einer V_{L}-Domäne verknüpft mit einer V_{H}-Domäne, wobei die Immunglobulin-Domäne spezifisch an ein Antigen bindet, das auf Tumorzellen exprimiert ist;
(b) einer zweiten Immunglobulin-Domäne bestehend aus einer V_{L}-Domäne verknüpft mit einer V_{H}-Domäne, wobei die Immunglobulin-Domäne spezifisch an ein Antigen bindet, das auf Tumorzellen exprimiert ist; und
(c) einer dritten Immunglobulin-Domäne bestehend aus einer V_{L}-Domäne verknüpft mit einer V_{H}-Domäne, wobei die Immunglobulin-Domäne spezifisch an ein Effektorzell-Antigen bindet, wobei die Effektorzelle ausgewählt ist aus der Gruppe bestehend aus NK-Zellen, T-Zellen, neutrophilen Granulozyten, Monozyten und Makrophagen,
wobei mindestens eines der Antigene, das auf Tumorzellen exprimiert ist und das von der Immunglobulin-Domäne nach (a) oder (b) gebunden wird, ein Antigen ist, das auf Tumorstammzellen und/oder Tumor-Vorstufen- und -Vorläuferzellen exprimiert ist; und
(d) gegebenenfalls mindestens einem (Poly)peptid, das unterschiedlich zu Immunglobulin-Domänen ist; besteht
und wobei das Nukleinsäuremolekül mindestens einen Linker kodiert, der mindestens eine V_{H}- von einer V_{L}-Domäne oder mindestens eine V_{L}- von einer V_{H}-Domäne trennt.

2. Nukleinsäuremolekül nach Anspruch 1, wobei die Immunglobulin-Domänen nach Punkten (a) und (b) an das gleiche Antigen, das auf Tumorzellen exprimiert ist, binden.

3. Nukleinsäuremolekül nach Anspruch 1, wobei die Immunglobulin-Domänen nach Punkten (a) und (b) an verschiedene Antigene, die auf Tumorzellen exprimiert sind, binden.

4. Nukleinsäuremolekül nach einem der Ansprüche 1 bis 3, wobei das mindestens eine Antigen, das auf Tumorstammzellen oder Tumor-Vorstufen- und -Vorläuferzellen exprimiert ist, CD19, CD33, CD13, CD44var, CD123, CD96 oder CLL-1 ist.

5. Nukleinsäuremolekül nach einem der Ansprüche 1 bis 4, wobei die Antigene, die auf Tumorzellen exprimiert und von den Immunglobulin-Domänen nach (a) und (b) gebunden werden CD19 und CD33, CD19 und CD13, CD19 und CD123, CD19 und CD44var, CD19 und MCSP, CD33 und CD13, CD33 und CD123, CD33 und CD44var, CD33 und CD96, CD33 und CLL-1, CD13 und CD123, CD13 und CD44var, CD13 und CD96, CD13 und CLL-1, CD123 und CD44var, CD123 und CD96, CD123 und CLL-1, CD44var und CD96, CD44var und CLL-1, oder CD96 und CLL-1 sind.

6. Nukleinsäuremolekül nach einem der Ansprüche 1 bis 5, wobei das Effektorzell-Antigen, das von der Immunglobulin-Domäne nach (c) gebunden wird, CD16 (FcγRIIIa), CD64 (FcγRI), NKG2D, NKp30, NKp44, NKp46, CD28, CD2 oder CD3 ist.

7. Nukleinsäuremolekül nach einem der Ansprüche 1 bis 6, wobei mindestens eine Immunglobulin-Domäne mindestens einen Cystein-Rest umfasst, der intramolekulare Disulfidbrücken ausbilden kann.

8. Nukleinsäuremolekül nach einem der Ansprüche 1 bis 7, wobei der Linker ein Peptidlinker mit mindestens 5 Aminosäuren ist.

9. Nukleinsäure nach Anspruch 8, wobei die Linker eine einheitliche Länge haben, wenn mehr als ein Linker vorhanden ist.

10. Nukleinsäure nach Anspruch 8, wobei die Linker eine unterschiedliche Länge haben, wenn mehr als ein Linker vorhanden ist.

11. Nukleinsäure nach Anspruch 9, wobei die Aminosäuresequenzen der Linker identisch sind.

12. Nukleinsäure nach Anspruch 9, wobei die Aminosäuresequenzen der Linker unterschiedlich sind.

13. Nukleinsäure nach einem der Ansprüche 1 bis 12, die zwei Immunglobulin-Domänen, die spezifisch an CD19 binden, und eine Immunglobulin-Domäne, die spezifisch an CD16 (FcγRIIIa) bindet, kodiert.

14. Nukleinsäure nach einem der Ansprüche 1 bis 12, die zwei Immunglobulin-Domänen, die spezifisch an CD33 binden, und eine Immunglobulin-Domäne, die spezifisch an CD16 (FcγRIIIa) bindet, kodiert.

15. Nukleinsäuremolekül nach Anspruch 1, wobei das (Poly)peptid, das unterschiedlich zu Immunglobulin-Domänen ist, ein Strep-Tag, ein His-Tag, ein Myc-Tag, ein Flag-Tag, ein Kappa-Sekretions-Signal, menschliches Serumalbumin (hsa) oder Fragmente davon, Peptide, die an hsa oder andere Serumproteine binden können, ein Peptid, das an den neonatalen Fc-Rezeptor (FcRn) binden kann, menschliche Muskel-Aldolase (hma) oder ein Fragment davon, CD8-Gelenk("Hinge")-Region, Interleukin-2, Interleukin-15, Interleukin-18, Granulozyten-Makrophagen-Kolonie-stimulierender Faktor (GM-CSF), Granulozyten-Kolonie-stimulierender Faktor (G-CSF) oder ein Peptid, das mindestens eine N-Glykosylierungsstelle bereitstellt, ist/sind.

16. Nukleinsäuremolekül nach einem der Ansprüche 1 bis 15, wobei die Tumorzellen Leukämiezellen sind.

17. Nukleinsäuremolekül nach einem der Ansprüche 1 bis 15, wobei die Tumorstammzellen leukämische Stammzellen sind.

18. Nukleinsäuremolekül nach einem der Ansprüche 1 bis 15, wobei die Tumor-Vorstufen- und -Vorläuferzellen leukämische Tumor-Vorstufen- und -Vorläuferzellen sind.

19. Nukleinsäuremolekül nach einem der Ansprüche 1 bis 18, wobei der Tumor eine Leukämie oder ein Lymphom ist.

20. Vektor, umfassend das Nukleinsäuremolekül nach einem der Ansprüche 1 bis 19.

21. Nicht-menschlicher Wirt, der mit dem Vektor nach Anspruch 20 transformiert ist.

22. Nicht-menschlicher Wirt nach Anspruch 21, wobei der Wirt eine Zelle ist.

23. Verfahren zur Herstellung eines Polypeptids umfassend das Züchten der Wirtszelle nach Anspruch 22 unter geeigneten Bedingungen und Isolierung des hergestellten rekombinanten Polypeptids.

24. Polypeptid, das durch das Nukleinsäuremolekül nach einem der Ansprüche 1 bis 19 kodiert wird oder das mit dem Verfahren nach Anspruch 23 hergestellt wird.

25. Diagnostische Zusammensetzung umfassend mindestens eines von
(a) dem Nukleinsäuremolekül nach einem der Ansprüche 1 bis 19
(b) dem Vektor nach Anspruch 20 oder
(c) dem Polypeptid nach Anspruch 24.

26. Pharmazeutische Zusammensetzung umfassend
(a) das Nukleinsäuremolekül nach einem der Ansprüche 1 bis 19
(b) den Vektor nach Anspruch 20 oder
(c) das Polypeptid nach Anspruch 24.

27. Nukleinsäuremolekül nach einem der Ansprüche 1 bis 19, Vektor nach Anspruch 20 oder Polypeptid nach Anspruch 24 zur Verwendung in der Behandlung von Tumoren.

28. Nukleinsäuremolekül oder Vektor oder Polypeptid zur Verwendung nach Anspruch 27, wobei der Tumor eine Leukämie oder ein Lymphom ist.

29. Nukleinsäuremolekül oder Vektor oder Polypeptid zur Verwendung nach Anspruch 28, wobei die Leukämie AML, CML, ALL, CLL oder Non-Hodgkin-Lymphom ist.

## Revendications

1. Molécule d'acide nucléique codant pour un polypeptide,
où le polypeptide est constitué
(a) d'un premier domaine d'immunoglobuline constitué d'un domaine VL lié à un domaine VH, où le domaine d'immunoglobuline se lie spécifiquement à un antigène exprimé sur des cellules tumorales ;
(b) d'un deuxième domaine d'immunoglobuline constitué d'un domaine VL lié à un domaine VH, où le domaine d'immunoglobuline se lie spécifiquement à un antigène exprimé sur des cellules tumorales ; et
(c) d'un troisième domaine d'immunoglobuline constitué d'un domaine VL lié à un domaine VH, où le domaine d'immunoglobuline se lie spécifiquement à un antigène de cellule effectrice, où la cellule effectrice est choisie dans le groupe constitué de cellules NK, lymphocytes T, granulocytes neutrophiles, monocytes et macrophages,
où au moins l'un des antigènes exprimés sur des cellules tumorales et liés par le domaine d'immunoglobuline de (a) ou (b) est un antigène exprimé sur des cellules souches tumorales et/ou des cellules précurseurs ou progéniteurs tumorales ; et
(d) éventuellement, d'au moins un (poly)peptide non apparenté à des domaines d'immunoglobulines ;
et où, la molécule d'acide nucléique code pour au moins un lieur qui sépare au moins un domaine V_{H} d'un domaine V_{L} ou au moins un domaine V_{L} d'un domaine V_{H}.

2. Molécule d'acide nucléique selon la revendication 1, où les domaines d'immunoglobuline des éléments (a) et (b) se lient au même antigène exprimé sur des cellules tumorales.

3. Molécule d'acide nucléique selon la revendication 1, où les domaines d'immunoglobuline des éléments (a) et (b) se lient à des antigènes différents exprimés sur des cellules tumorales.

4. Molécule d'acide nucléique selon l'une quelconque des revendications 1 à 3, où le au moins un antigène exprimé sur des cellules souches tumorales ou sur des cellules précurseurs ou progéniteurs tumorales est CD 19, CD33, CD 13, CD44var, CD123, CD96 ou CLL-1.

5. Molécule d'acide nucléique selon l'une quelconque des revendications 1 à 4, où les antigènes exprimés sur des cellules tumorales et liés par les domaines d'immunoglobulines de (a) et (b) sont CD19 et CD33, CD19 et CD13, CD19 et CD123, CD19 et CD44var, CD19 et MCSP, CD33 et CD13, CD33 et CD123, CD33 et CD44var, CD33 et CD96, CD33 et CLL-1, CD13 et CD123, CD13 et CD44var, CD 13 et CD96, CD 13 et CLL-1, CD123 et CD44var, CD123 et CD96, CD123 et CLL-1, CD44var et CD96, CD44var et CLL-1, ou CD96 et CLL-1.

6. Molécule d'acide nucléique selon l'une quelconque des revendications 1 à 5, où l'antigène de la cellule effectrice lié par le domaine d'immunoglobuline de (c) est CD16 (FcγRIIIa), CD64 (FcγRI), NKG2D, NKp30, NKp44, NKp46, CD28, CD2 ou CD3.

7. Molécule d'acide nucléique selon l'une quelconque des revendications 1 à 6, où au moins un domaine d'immunoglobuline comprend au moins un résidu de cystéine capable de former des ponts disulfure intramoléculaires.

8. Molécule d'acide nucléique selon l'une quelconque des revendications 1 à 7, où le lieur est un lieur peptidique d'au moins 5 acides aminés.

9. Acide nucléique selon la revendication 8, où les lieurs sont de longueur uniforme si plus d'un lieur est présent.

10. Acide nucléique selon la revendication 8, où les lieurs sont de longueur différente si plus d'un lieur est présent.

11. Acide nucléique selon la revendication 9, où les séquences d'acides aminés des lieurs sont identiques.

12. Acide nucléique selon la revendication 9, où les séquences d'acides aminés des lieurs sont différentes.

13. Acide nucléique selon l'une quelconque des revendications 1 à 12, codant pour deux domaines d'immunoglobulines se liant spécifiquement à CD 19 et un domaine d'immunoglobuline se liant spécifiquement à CD16 (FcγRIIIa).

14. Acide nucléique selon l'une quelconque des revendications 1 à 12, codant pour deux domaines d'immunoglobulines se liant spécifiquement à CD33 et un domaine d'immunoglobuline se liant spécifiquement à CD16 (FcγRIIIa).

15. Molécule d'acide nucléique selon la revendication 1, où le (poly)peptide non apparenté à des domaines d'immunoglobulines est un marqueur Strep, un marqueur His, un marqueur Myc, un marqueur Flag, une séquence leader de sécrétion de chaîne kappa, la sérumalbumine humaine (hsa) ou des fragments de celle-ci, des peptides capables de se lier à la hsa ou à d'autres protéines sériques, un peptide capable de se lier au récepteur Fc néonatal (FcRn), l'aldolase musculaire humaine (amh) ou un fragment de celle-ci, la région charnière de CD8, l'interleukine 2, l'interleukine 15, l'interleukine 18, le facteur de stimulation des colonies de granulocytes et de macrophages (GM-CSF), le facteur de stimulation des colonies de granulocytes (G-CSF) ou un peptide fournissant au moins un site de N-glycosylation.

16. Molécule d'acide nucléique selon l'une quelconque des revendications 1 à 15, où les cellules tumorales sont des cellules leucémiques.

17. Molécule d'acide nucléique selon l'une quelconque des revendications 1 à 15, où les cellules souches tumorales sont des cellules souches leucémiques.

18. Molécule d'acide nucléique selon l'une quelconque des revendications 1 à 15, où les cellules précurseurs ou progéniteurs tumorales sont des cellules précurseurs ou progéniteurs leucémiques.

19. Molécule d'acide nucléique selon l'une quelconque des revendications 1 à 18, où la tumeur est une leucémie ou un lymphome.

20. Vecteur comprenant la molécule d'acide nucléique selon l'une quelconque des revendications 1 à 19.

21. Hôte non humain transformé avec le vecteur selon la revendication 20.

22. Hôte non humain selon la revendication 21, où l'hôte est une cellule.

23. Méthode de production d'un polypeptide comprenant la culture de la cellule hôte selon la revendication 22 dans des conditions appropriées et l'isolement du polypeptide recombinant produit.

24. Polypeptide codé par la molécule d'acide nucléique selon l'une quelconque des revendications 1 à 19 ou produit par la méthode selon la revendication 23.

25. Composition de diagnostic comprenant au moins l'un
(a) de la molécule d'acide nucléique selon l'une quelconque des revendications 1 à 19
(b) du vecteur selon la revendication 20 ou
(c) du polypeptide selon la revendication 24.

26. Composition pharmaceutique comprenant
(a) la molécule d'acide nucléique selon l'une quelconque des revendications 1 à 19
(b) le vecteur selon la revendication 20 ou
(c) le polypeptide selon la revendication 24.

27. Molécule d'acide nucléique selon l'une quelconque des revendications 1 à 19, vecteur selon la revendication 20 ou polypeptide selon la revendication 24 pour une utilisation dans le traitement de tumeurs.

28. Molécule d'acide nucléique ou vecteur ou polypeptide pour une utilisation selon la revendication 27, où la tumeur est une leucémie ou un lymphome.

29. Molécule d'acide nucléique ou vecteur ou polypeptide pour une utilisation selon la revendication 28, où la leucémie est une LMA, une LMC, une LLA, une LLC ou un lymphome non hodgkinien.
